(19) Europäisches Patentamt
European Patent Office
Office européen des brevets

(11) Publication number: 0 310 824 B1

# (12) EUROPEAN PATENT SPECIFICATION

(45) Date of publication of patent specification: 10.08.94 (51) Int. Cl.$^5$: C12M 1/40

(21) Application number: 88114860.5

(22) Date of filing: 12.09.88

## (54) Method and apparatus for the determination of two different substances in a sample using enzyme electrodes.

(30) Priority: 11.09.87 JP 228730/87

(43) Date of publication of application:
12.04.89 Bulletin 89/15

(45) Publication of the grant of the patent:
10.08.94 Bulletin 94/32

(84) Designated Contracting States:
DE GB

(56) References cited:

ANALYTICAL BIOCHEMISTRY, vol. 149, 1985, Academic Press Inc., Baltimore, MD (US); T. YAO et al., pp. 387-391&NUM;

PATENT ABSTRACTS OF JAPAN, vol. 10, no. 271 (P-497)[2327], 16 September 1986&NUM;

ANALYTICA CHIMICA ACTA, vol. 171, 1985, Elsevier Science Publishers BV, Amsterdam (NL); M. MASOOM et al., pp. 185-194&NUM;

BIOCHIMIE, no. 62, 1980, Paris (FR); D. PFEIFFER et al., pp. 587-593&NUM;

(73) Proprietor: NEW OJI PAPER CO., LTD.
7-5, Ginza 4-chome,
Chuo-ku
Tokyo (JP)

(72) Inventor: Hayashi, Ryuzo
7-26, Hishiyanishi 5-chome
Higashiosaka-shi Osaka, 577 (JP)
Inventor: Kariyone, Akio
50-9, Ishiicho
Nishishichijo
Shimogyo-ku
Kyoto-shi Kyoto, 600 (JP)
Inventor: Hashizume, Yoshio
2-16, Minamishowacho
Nishinomya-shi Hyogo-ken, 662 (JP)

(74) Representative: Patentanwälte Beetz - Timpe - Siegfried Schmitt-Fumian - Mayr
Steinsdorfstrasse 10
D-80538 München (DE)

EP 0 310 824 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a flow-injection method and a flow injection apparatus for the quantitative determination, i.e. the measurement of the concentration, of two different substances in a liquid sample by means of two enzyme electrodes detecting ultimately the same material, wherein on one of these enzyme electrodes a first substance to be measured is yield from a second substance by enzymatic reaction.

In recent years, immobilized enzymes have been broadly applied, e.g. in the fields of clinical test, enzyme production, fermentation technology and analytical chemistry. Especially, immobilized enzyme electrodes are widely utilized because of their high selectivity, readiness and easiness of measuring.

Recently, apparatus for simultaneously measuring two different substances were introduced, which measure simultaneously for instance

- glucose and uric acid (JP-A1-24142/1987);
- lactic acid and pyruvic acid (JP-A1-5172/1987);
- glucose and lactic acid, or glucose and choline (GB 2063479);
- sucrose and glucose (Analytica Chimica Acta, 171 (1985), 185-194)

According to this prior art, however, each enzyme immobilized on the electrode catalyzes a different reaction, respectively.

On the contrary, in the case of common reactions which yield or consume the electrode active material (for example, ultimately hydrogen peroxide is formed, and the amount of the hydrogen peroxide is measured), the concentration of a first substance (e.g. glucose) can be measured by one electrode, but the other electrode detects not only hydrogen peroxide originating from the second substance (e.g. sucrose) but also hydrogen peroxide originating from the first substance (glucose). So the first substance (glucose) can be measured, but the result of measurement of the other corresponds to the sum of the two substance values.

For example, in the case of measuring the sucrose concentration in a sample containing both glucose and sucrose using enzyme electrodes, the result is affected by the glucose concentration:

(i) $$\text{sucrose} + H_2O \xrightarrow{\text{invertase}} \alpha\text{-D-glucose} + \text{fructose}$$

(ii) $$\alpha\text{-D-glucose} \xrightarrow{\text{mutarotase}} \beta\text{-D-glucose}$$

(iii) $$\beta\text{-D-glucose} + O_2 \xrightarrow{\text{glucose oxidase}} H_2O_2 + \text{gluconic acid}$$

Generally in order to measure the sucrose concentration, sucrose is hydrolyzed to fructose and α-D-glucose by invertase (equation (i)). Thereafter α-D-glucose is converted to β-D-glucose by mutarotase (equation (ii)), and hydrogen peroxide, that is the electrode active material, is formed as a result of the oxidation of β-D-glucose catalyzed by glucose oxidase (equation (iii)). This hydrogen peroxide is detected electrochemically by the enzyme electrode (C. Bertrand, P.R. Coulet, D.C. Gautheron, Anal. Chim. Acta 126 (1981), 23-34). However according to this method, the enzyme electrode measures not only β-D-glucose converted from sucrose according to equations (i) and (ii), but also β-D-glucose originally contained in the sample, because β-D-glucose originally contained in the sample yields hydrogen peroxide according to equation (iii) as well as β-D-glucose formed according to equations (i) and (ii) does.

Therefore, in order to measure the correct concentration of only sucrose, it is necessary to provide a layer containing glucose oxidase and catalase, in which glucose is oxidized by the glucose oxidase and hydrogen peroxide is removed by the catalase enzymatically before measuring sucrose (F. Scheller, R.Renneberg, Anal. Chim. Acta 152 (1983), 265-269), or to employ an enzymatic reaction and an electrochemical technique in order to avoid the influence of glucose originally contained in the sample (JP-A1-216947/1983).

But the apparatus employing the above described method have some drawbacks in that it is extremely difficult to immobilize the enzymes to the membranes in preparing the enzyme electrodes and that the electrodes themselves are very complicated. Furthermore, in order to measure two different substances, that is e.g. glucose and sucrose, another electrode must be provided for measuring the glucose concentration by this electrode.

Recently, a flow type measuring apparatus was reported, which comprises a column reactor wherein a sample solution is circulated (M. Massoom, A. Townshend, Anal. Chim. Acta 171 (1985), 185-194)). This apparatus, however, is complicated, and a far higher amount of enzyme is needed as compared with the apparatus having enzyme electrodes. Furthermore, it also has the drawback that it gives uncorrect results because of choking of the column.

The case of measuring samples containing both sucrose and glucose is described above, however, when the same electrode active material is formed by the common enzymatic reaction on the two enzyme electrodes, in measuring two different substances simultaneously, for example in measuring maltose and glucose, or ester type cholesterol and free cholesterol, the same kinds of problems are caused.

So it could be considered to measure the concentrations of two different substances, for example glucose as the first substance to be measured and sucrose as the second substance to be measured, using two enzyme electrodes by means of a flow type measuring apparatus provided with a first enzyme electrode having immobilized glucose oxidase for measuring glucose, and a second enzyme electrode having immobilized glucose oxidase, mutarotase and invertase for measuring sucrose.

The glucose concentration detected by the first enzyme electrode is calculated, and the correct concentration of only sucrose is obtained by subtracting the result of the calculation from the output current value of the second enzyme electrode.

According to this method, however, when the glucose concentration of the sample is comparatively high, because of converting sucrose to glucose on the second enzyme electrode, a great amount of the electrode active material is formed. Therefore, the amount exceeds the range, in which the output current is proportional to the amount of the electrode active material. So accurate values of the sucrose concentration cannot be obtained.

In this case, by using a second enzyme electrode having a reduced amount of immobilized enzymes and lower sensitivity, samples of high substances concentration can be analyzed. On the other hand, in this method, electrodes of different sensitivity corresponding to the concentrations of the substances in the sample solution must be prepared. So the apparatus employing this method is not practical at all.

It is the object of the invention to provide a method and an apparatus for the measurement, i.e. the quantitative concentration determination, of a plurality of substances in a liquid sample by means of enzyme electrodes which give accurate concentration values in a short time.

The above object is accomplished according to claims 1 and 5. The dependent claims relate to preferred embodiments.

The flow injection method of the present invention for the quantitative determination of a substance (second substance) which is capable of being enzymatically converted into another substance (first substance), contained in a liquid sample by means of two enzyme electrodes which detect the first substance, the second enzyme electrode catalyzing the conversion of the second substance into the first substance prior to its detection, comprises the following steps:

(I) Determining the response ratio k of the two enzyme electrodes regarding the first substance by

(A) supplying a constant flow of a buffer solution,

(B) injecting the first substance into the flow of the buffer solution,

(C) passing the solution obtained in step (B) through a first measuring cell comprising the first enzyme electrode for detecting the first substance and measuring the output current ($I_a$),

(D) passing the solution after step (C) through a dilution pipeline, and

(E) passing the solution after step (D) through a second measuring cell comprising the second enzyme electrode for detecting the first substance, and measuring the output current ($I_b$),

(II) calculating the response ratio k according to the equation $\alpha$,

$$k = I_b/I_a \qquad (\alpha),$$

(III) Selecting a suitable length of the dilution pipeline according to the concentration ratio of the first and the second substance,

(IV) determining the second substance by

(I) injecting the sample to be analyzed containing the first substance and the second substance into the flow of the buffer solution,

(G) passing the solution obtained in step (I) through the first measuring cell comprising the first enzyme electrode and measuring the output current ($I_1$),

(H) passing the solution after step (G) through the dilution pipeline,

(J) passing the solution after step (H) through the second measuring cell comprising the second enzyme electrode adapted to convert the second substance to be determined into the first substance, and measuring the output current ($I_2$),

and

(V) calculating the current value $I_3$ corresponding to the amount of the second substance to be determined according to the equation ($\beta$),

$$I_3 = I_2 - k \cdot I_1 \qquad (\beta),$$

wherein k is the response ratio of the two enzyme electrodes determined in step (II).

According to a preferred embodiment, also the first substance is quantitatively determined from the output current ($I_1$) of the first measuring cell measured in step (G) and corresponding to the amount of the first substance.

Preferably, steps (I) (B) to (V) are carried out intermittently.

The flow injection apparatus of the present invention for the quantitative determination of a substance (second substance) which is capable of being enzymatically converted into another substance (first substance), contained in a liquid sample by means of two enzyme electrodes, which is particularly adapted for carrying out the method according to the invention,

comprises

- supply means for continuously supplying a buffer solution at a predetermined flow rate,
- injection means for injecting samples to be measured into the flow of the buffer solution,
- a first measuring cell comprising a first enzyme electrode for detecting the first substance and being provided downstream of the injecting means,
- a dilution pipeline provided downstream of the first measuring cell for diluting the sample along its flow direction,
- a second measuring cell provided at the downstream end of the dilution pipeline comprising a second enzyme electrode having an enzyme immobilized thereon for catalyzing the conversion of the second substance into the first substance and for detecting (a) the first substance originally contained in the sample and (b) the first substance resulting from the conversion of the second substance,
- means for calculating the response ratio k according to the equation $\alpha$,

$$k = I_b/I_a \qquad (\alpha),$$

wherein

$I_a$ is the measured output current value of the first measuring cell,

and

$I_b$ is the measured output current value of the second measuring cell,

and for calculating the current value $I_3$ corresponding to the concentration of the second substance to be determined according to the equation $\beta$,

$$I_3 = I_2 - k \cdot I_1 \qquad (\beta),$$

wherein

$I_1$ is the output current of the first measuring cell, and

$I_2$ is the output current of the second measuring cell,

and

- means for selecting a suitable length of the dilution pipeline according to the concentration ratio of the first and second substance, the apparatus being adapted to carry out the method steps (I) to (V) as defined above.

In accordance with the preferred embodiments, the apparatus of the present invention is characterized by one or more of the following features:

- The injection means are a HPLC injector;
- the dilution pipeline has an inner diameter of the flow path of 0,1 to 2,0 mm and an axial length of 0,2 to 100 m;
- the dilution pipeline is made of a synthetic resin;
- the dilution pipeline is made of stainless steel;
- the dilution pipeline is coiled;

- it comprises a plurality of dilution pipelines, each having a different inner diameter and/or a different axial length, respectively, and switching valves are provided to selectively connect one of the dilution pipelines between the first measuring cell and the second measuring cell.

According to a preferred embodiment of the invention, the means for calculating the response ratio k according to equation (α) and the current value $I_3$ according to equation (β) are adapted to determine also the amount of the first substance from the output current ($I_1$) of the first measuring cell measured in step (G). Furthermore, these means are preferably adapted to control the injection means for intermittently, successively or independently carrying out the above described steps (I) β to (V).

The following combinations correspond to preferred embodiments of the apparatus according to the invention:

- Where the first substance is glucose, and the second substance is sucrose, the first enzyme electrode has glucose oxidase immobilized thereon, and the second enzyme electrode has glucose oxidase, mutarotase and invertase immobilized thereon;
- where the first substance is glucose and the second substance is maltose, the first enzyme electrode has glucose oxidase immobilized thereon, and the second enzyme electrode has glucose oxidase, mutarotase and α-glucosidase immobilized thereon;
- where the first substance is glucose, and the second substance is a β-glucoside, the first enzyme electrode has glucose oxidase immobilized thereon, and the second enzyme electrode has glucose oxidase and β-glucosidase immobilized thereon;
- where the first substance is glucose, and the second substances are maltooligosugars, the first enzyme electrode has glucose oxidase immobilized thereon, and the second enzyme electrode has glucose oxidase and glucoamylase immobilized thereon;
- where the first substance is glucose, and the second substance is lactose, the first enzyme electrode has glucose oxidase immobilized thereon, and the second enzyme electrode has glucose oxidase and lactase immobilized thereon, and
- where the first substance is free cholesterol, and the second substance is a cholesteryl ester, the first enzyme electrode has cholesterol oxidase immobilized thereon, and the second enzyme electrode has cholesterol oxidase and cholesterol esterase immobilized thereon.

According to the concept of this invention, the sample is injected in the flow-type measuring apparatus into a constant flow of buffer solution, and the enzyme electrodes of the two measuring cells are in contact with the liquid flow through the dilution pipeline situated downstream of the first enzyme electrode, and the second enzyme electrode situated downstream of the stream, for detecting for example sucrose, as a second substance, and glucose. Therefore, by the dilution pipeline the sample is automatically diluted, and the second enzyme electrode is able to be responsive to higher concentrations of the substances proportionally. So the concentrations of the plural kinds of substances can be measured accurately.

In accordance therewith, the concept of the invention allows quick, easy and accurate measurements of plural kinds of substances.

In the following, the invention will be described in more detail with reference to the drawings:

Fig. 1 is a system diagram showing a measuring apparatus corresponding to one embodiment of the invention;

Fig. 2 is a graph showing the response curve of the second enzyme electrode in response to glucose according to the invention;

Fig. 3 is a graph showing the response curve of the second enzyme electrode in response to glucose without using the dilution pipeline ;

Fig. 4 is a system diagram showing another embodiment of the apparatus according to the invention;

Fig. 5 is a graph showing the output current values of the first and second enzyme electrodes in response to glucose and sucrose in Example 1;

Fig. 6 is a graph showing the output current values of the first and second enzyme electrodes in response to a sample of 30 mM glucose in dependence of the concentration of sucrose;

Fig. 7 is a graph showing a calibration curve for sucrose in accordance with Example 1;

Fig. 8 is a graph showing the output current values of the first and the second enzyme electrodes in response to glucose in dependence of the glucose concentration without using the dilution pipeline in accordance with Comparative Example 1;

Fig. 9 is a graph showing the output current values of the first and second enzyme electrodes in response to glucose and maltose in accordance with Example 2;

Fig. 10 is a graph showing the output current values of the first and second enzyme electrodes in response to a sample of 30 mM glucose in dependence of the concentration of maltose according to Example 2;

Fig. 11 is a graph showing a calibration curve for maltose in accordance with Example 2;

Fig. 12 is a graph showing the output current values of the first an second enzyme electrodes in response to glucose without using the dilution pipeline in accordance with Comparative Example 2;

Fig. 13 is a graph showing the output current values of the first enzyme electrode in response to glucose according to Example 3;

Fig. 14 is a graph showing the output current values of the second enzyme electrode in response to glucose according to Example 3;

Fig. 15 is a graph showing a calibration curve for lactose in accordance with Example 3, and

Fig. 16 is a graph showing the output current values of the second enzyme electrode in response to glucose without using the dilution pipeline in accordance with Comparative Example 3.

In the following, the general concept of the invention will be described in a detailed manner with reference to preferred embodiments.

Fig. 1 shows a measuring apparatus according to the invention. A buffer solution 8 stored in a vessel 7 is supplied through a pump 6 at a predetermined flow rate. The buffer solution from the pump 6 is caused to flow into the first measuring cell 3 through the injection means 1. For example, an injector, which is generally used in high performance liquid chromatography, is preferably used as injection means 1. The samples to be introduced through the injection means 1 contain two substances, i.e., a first substance to be measured (for example, glucose) and a second substance to be measured (for example, sucrose). A first enzyme electrode 2 and a reference electrode 10 are disposed facing each other across the flow path 9 formed in the first measuring cell 3. A dilution pipeline 5 is connected to the downstream side of the first measuring cell 3. The dilution pipeline 5 preferably has a flow path of an inner diameter in the order of 0,1 to 2,0 mm and an axial length in the order of 0,2 to 10 m, so that the first and second substances to be measured are prevented from being diffused and are diluted in the buffer solution over an excessively wide range in the axial direction of the dilution pipeline 5; thus it is possible to perform an effective dilution at high speed. The axial length of the dilution pipeline 5 is suitably adjusted according to the concentration ratio of the first substance and the second substance to be measured. If the length is too short, no sufficient effect of dilution can be obtained, and if it is too long, considerable time is required for measurement. Therefore, the axial length of te dilution pipeline 5, as indicated above, should be preferably of the order of 0,2 to 10 m and more preferably 0,5 to 3 m. The dilution pipeline 5 may be made of a fluoric resin, such as poly(trafluoroethylene)(PTFE Teflon®) or other synthetic resins such as polyvinyl chloride, or may be made of a metallic material, such as stainless steel. In order to allow an efficient mixing of the first and second substances with the buffer solution, the dilution pipeline 5 is configured in the form of a coil.

The second measuring cell 11 is connected to the downstream end of the dilution pipeline 5. In the second measuring cell 11, a second enzyme electrode 4 and a reference electrode 12 are disposed in face-to-face relation to each other across the flow path 13. In order to prevent a wide variation of the measured peak values at the enzyme electrodes 2, 4, the capacity of the flow paths 9, 13 of the measuring cells 3, 11 is preferably of the order of 5 to 100 µl.

The first enzyme electrode 2 detects the first substance.

The second enzyme electrode 4 exhibits enzyme reactions to produce the first substance from the second substance and also detects the first substance.

Where the first substance is glucose, and the second substance is sucrose, the first enzyme electrode 2 is an electrode having glucose oxidase immobilized thereon, and the second enzyme electrode 4 is an electrode having glucose oxidase, mutarotase and invertase immobilized thereon.

Where the first substance is glucose, and the second substance is maltose, the first enzyme electrode 2 is an electrode having glucose oxidase immobilized thereon, and the second enzyme electrode 4 is an electrode having glucose oxidase, mutarotase and $\alpha$-glucosidase immobilized thereon.

Where the first substance is glucose, and the second substance is a $\beta$-glucoside, the first enzyme electrode 2 is an electrode having glucose oxidase immobilized thereon, and the second enzyme electrode 4 is an electrode having glucose oxidase and $\beta$-glucosidase immobilized thereon.

Where the first substance is glucose, and the second substances are maltooligosugars, the first enzyme electrode 2 is an electrode having glucose oxidase immobilized thereon, and the second enzyme electrode 4 is an electrode having glucose oxidase and glucoamylase immobilized thereon.

Where the first substance is glucose, and the second substance is lactose, the first enzyme electrode 2 is an electrode having glucose oxidase immobilized thereon, and the second enzyme electrode 4 is an electrode having glucose oxidase and lactase immobilized thereon.

For detecting free cholesterol and esterified cholesterol, the first enzyme electrode 2 is an electrode having cholesterol oxidase immobilized thereon, and the second enzyme electrode 4 is an electrode having cholesterol oxidase and cholesterol esterase immobilized thereon.

A characteristic feature of the present invention is that, after measurement of the first substance by the first enzyme electrode 2, the sample leaving the first measuring cell 3, that is a solution of the first and/or the second substance in the buffer solution, is guided into the dilution pipeline 5 so that the first and second substances are diffused and diluted in the buffer solution within the dilution pipeline 5. For example, when glucose as said first substance to be measured is injected through the injection means 1, and the same is detected by the second enzyme electrode 4, a response curve as shown in Fig. 2 can be obtained, in which the maximal output current value from the second enzyme electrode 4 is comparatively small, so that first and second substances of high concentration may effectively be measured. In contrast, if a buffer solution containing the first and/or the second substance to be measured is guided directly into the second measuring cell 11 without using the dilution pipeline 5, the maximal output current value from the second enzyme electrode 4 is considerably high as shown in Fig. 3. As a consequence, the concentration of the first substance detected by the second enzyme electrode 4 is outside the upper limit of its proportional range, and thus it is practically impossible to perform an accurate measurement of the second substance. The dilution pipeline 5 eliminates this problem.

Generally, the proportional range of the concentration of a substance to be measured in relation to the value of the output current from an enzyme electrode is determined by kinetic constants and the diffusion coefficient of the substance to be measured in the immobilized enzyme layer, and, where oxygen is required, as it is the case with immobilized oxidase, the amount of dissolved oxygen.

Therefore, if the concentration of the first substance as measured by the second enzyme electrode 4 is excessively high, it is likely to deviate from the aforesaid proportional range, which does not allow to perform an accurate measurement of the concentration of the first substance.

Fig. 4 shows another embodiment of the invention, which is applicable in cases where the first and second substances to be measured are considerably different in their concentrations, wherein a plurality of dilution pipelines 5, 5a (two in number in Fig. 4) are provided, so that they may be changed over from one to the other by means of the switching valves 14, 15. The dilution pipelines 5, 5a are different in their dimensions, such as their flow path's Inner diameter and axial length. The dilution pipelines 5, 5a may be adapted to be interchangeably connected to the measuring cells 3, 11 by means of pipe joints, instead of being provided with the switching valves 14, 15.

In the measuring apparatus according to the invention, as shown in Fig. 1, while the buffer solution 8 is supplied by the pump 6 at a constant flow rate, the first substance to be measured is intermittently injected by the injection means 1 into the buffer solution. In this case, the first enzyme electrode 2 exhibits a response characteristic as indicated by line $\ell 1$ in Fig. 5, and the second enzyme electrode 4 gives a response characteristic as indicated by line $\ell 2$ in Fig. 5. Where, whithin the proportional range of the concentration of the first substance at the first and second enzyme electrodes 2, 4 in relation to the output current values from the electrodes, when the molarity of the first substance is represented by Ma, the output current value of the first enzyme electrode 2 is represented by $I_a$, and the output current value of the second enzyme electrode 4 is represented by $I_b$, the response ratio k is calculated according to the equation $\alpha$,

$$k = I_b/I_a \qquad (\alpha).$$

Next, samples of the first substance of a predetermined concentration mixed with the second substance of varied concentrations are intermittently introduced by the injection means 1. As a consequence, the first enzyme electrode 2 exhibits a response characteristic as indicated by the line $\ell 4$ in Fig. 6, and the second enzyme electrode 4 exhibits a response characteristic as indicated by the line $\ell 5$ in Fig. 6. In Fig. 6, the output $I_1$ of the first enzyme electrode 2 is represented by $\ell 4$, and the output $I_2$ of the second enzyme electrode 4 is represented by $\ell 5$. The output current $I_3$ of the second enzyme electrode 4 due to the second substance only is as shown by line $\ell 6$ in Fig. 7, which is expressed by formula $\beta$. The output current $I_3$ is proportional to the concentration of the second substance:

$$I_3 = I_2 \cdot k \cdot I_1 \qquad (\beta).$$

According to the invention, as above described, an automatic dilution of the samples takes place in the dilution pipeline 5 between the two enzyme electrodes 2 and 4, so that the proportional range at the second enzyme electrode can be extended. It is thus possible to measure samples of varied concentrations with good accuracy and in short time. Unlike apparatus based on the flow injection technique using columns, the measuring apparatus of the invention does not require any change of the flow path, and therefore it has the advantage that changes in the individual flow rates due to variations in back pressure or joint portion dead

capacity need not be considered.

In the following, the invention will be explained with reference to examples.

Example 1

In an apparatus according to Fig. 1, as the first enzyme electrode 2, a glucose detecting electrode having glucose oxidase immobilized thereon is employed, and for the second enzyme electrode 4, a glucose and sucrose detecting electrode having glucose oxidase, invertase and mutarotase immobilized thereon is employed.

Preparation of Enzyme Electrodes 2, 4

On the surface of a thoroughly cleaned platinum wire electrode having a diameter of 2 mm were put dropwise 5 µl of an aqueous solution containing 3 mg/ml of glucose oxidase (Type II, Sigma), 7 mg/ml of bovine serum albumin ("Fraction V", Sigma), and 0,5 mass-% of glutaraldehyde. Through a treatment of 30 min at 40 °C glucose oxidase was immobilized onto the surface. This electrode was employed as first enzyme electrode 2.

On the surface of a platinum electrode were put dropwise 5 µl of an aqueous solution containing 3 mg/ml of glucose oxidase, 1.5 mg/ml of invertase (grade X, Candida utilis, Sigma), 0,1 mg/ml of mutarotase (Sigma), 5,4 mg/ml of bovine serum albumin, and 0,5 mass-% of glutaraldehyde. By means of a treatment of 30 min at 40 °C, the enzymes were immobilized onto the surface. This electrode was employed as second enzyme electrode 4.

The measuring apparatus employed (Fig. 1) was of such an arrangement that an injector, which is generally used for high performance liquid chromatography and which is capable of injecting microliter samples, was used as injection means 1. The first measuring cell 3 having the first enzyme electrode 2 mounted therein was connected to the injection means 1 by a PTFE tube having an inner diameter of 0,5 mm and a length of 1,5 m. The measuring cell 3 was connected with the measuring cell 11 comprising the second enzyme electrode 4 by a PTFE-made dilution pipeline 5 of spiral form, with an outer diameter of 2,8 cm, having an extended length of 2,0 m and an inner diameter of 0,5 mm. The measuring cells 3, 11 each had an internal volume of 40 µl, and in their interior there were disposed Ag/AgCl reference electrodes 10, 12, respectively, facing the enzyme electrodes 2, 4. To each of the enzyme electrodes 2, 4 was applied a voltage of +0,45 V with respect to the corresponding Ag/AgCl reference electrode. For supplying a buffer solution, a high performance liquid chromatography pump was used as pump 6, and a buffer solution of 0,1M sodium phosphate and pH 7,0 was supplied at a flow rate of 1.0 ml/min.

In this system, aqueous solutions of glucose only (within the concentration range of 5 to 50 mM) and an aqueous solution of sucrose only (within the concentration range of 5 to 50 mM) were first injected in an amount of 5 µl each, and thus individual calibration curves ℓ1 - ℓ3 were obtained, as earlier described with reference to Fig. 5. With the first enzyme electrode 2, the linear range for glucose was up to 30 mM. Next, sucrose samples of varied concentrations (within the concentration range of 5 to 50 mM) each containing 30 mM of glucose were prepared, and calibration curves ℓ4, ℓ5 were obtained, as shown in Fig. 6. The response ratio k of the first enzyme electrode 2 and the second enzyme electrode 4 for glucose was determined from the calibration curves ℓ1, ℓ2 (in Fig. 5, the ratio between the output of the first enzyme electrode 2 and the output of the second enzyme electrode 4 for glucose is 0,46), by which the calibration curve ℓ5 in Fig. 6 for glucose-added sucrose was corrected as shown in Fig. 7. Thus, the calibration curve ℓ6 In Fig. 7 for sucrose which is free from the influence of glucose was obtained from the outputs of the first and second enzyme electrodes 2, 4. The calibration curve ℓ6 agreed with the calibration curve 13 In Fig. 5.

Whereas, in Fig. 6, the output $I_2$ of the second enzyme electrode 4 with respect to glucose and sucrose is represented by ℓ5, and the output $I_1$ of the first enzyme electrode 2 with respect to glucose is represented by ℓ4, Fig. 7 shows the corresponding calibration curve for the output $I_3$ relative to the sucrose concentration, that is, the values for $I_2 - 0{,}46 \bullet I_1$; with this curve, the value of the sucrose concentration can be determined. Even if the second enzyme electrode is in contact with 30 mM of glucose, its linear detection range for sucrose is up to 50 mM, from which fact it can be seen that even samples of high concentration are well detectable.

Comparative Example 1

The measurement was carried out in the same manner as in Example 1, but without using the dilution pipeline 5 in Fig. 1. The calibration curves ℓ7 of the electrodes 2, 4 for glucose showed a tendency for saturation (deviation from linearity) at about 30 mM, as may be seen from Fig. 8. That is, with samples containing glucose of 30 mM and sucrose, the outputs of the second enzyme electrode were outside the linear range and, therefore, no accurate measurement was possible.

Example 2

For the enzyme electrode 2 in Fig. 1, a glucose detecting electrode having glucose oxidase immobilized thereon was employed, and for the second enzyme electrode 4, a glucose and maltooligosugars detecting electrode having glucose oxidase and glucoamylase (Rhizopus, Sigma) immobilized thereon was employed. The first enzyme electrode was of the same construction as that used in Example 1. The second enzyme electrode 4 was prepared in the same manner as in Example 1, with use of the different kinds of enzymes. The other arrangement was the same as in Example 1.

According to the same procedure as used in Example 1, an aqueous solution of glucose only was first introduced through the injection means 1, and a calibration curve as shown by line ℓ8 in Fig. 9 was obtained. A calibration curve as shown by line ℓ9 was obtained from the second enzyme electrode 4. Then, maltose only was introduced through the injection means 1, and a calibration curve shown by ℓ10 was obtained.

Maltose solutions (within a maltose concentration range of 5 to 100 mM) containing 30 mM of glucose were prepared, and the response characteristic as shown by the line ℓ11 in Fig. 10 was obtained with the first enzyme electrode 2, and the response characteristic as shown by line ℓ12 with the second enzyme electrode 4. From these characteristics and according to the foregoing equations $\alpha$ and $\beta$ it is possible to obtain a calibration curve ℓ13 indicating the relationship between the concentration of maltose and the output current value of the second enzyme electrode 4 due to maltose only, as shown in Fig. 11.

Comparative Example 2

The measurement was carried out as in Example 2, whithout using the dilution pipeline 5. A calibration curve as shown by line ℓ14 in Fig. 12 was obtained. The output currents of the first and second enzyme electrodes 1 and 2 showed a tendency for saturation at a glucose concentration of about 30 mM. Therefore, with samples containing glucose of 30 mM and maltose, the output of the second enzyme electrode deviated from its measurable proportional range, and it was impossible to perform an accurate measurement of the maltose concentration.

Example 1, Comparative Example 2, and Comparative Example 2 may be summarized as follows. In Example 1, the glucose concentration can be measured on the basis of the output current values of the first enzyme electrode 2 in Fig. 1. Even with samples containing glucose of 30 mM, it is possible to measure the sucrose concentration from the output current values of the first and second enzyme electrodes 2, 4 as shown in Fig. 7. In Example 2, the glucose concentration can be measured from the output current value of the first enzyme electrode 2 in Fig. 9, and even if the samples contain glucose of 30 mM, it is possible to measure the maltose concentration from the output current values of the first and second enzyme electrodes as shown in Fig. 11. In contrast, it is apparent that in Comparative Examples 1 and 2, if the samples contain glucose of 30 mM, the upper limit of the linear measuring range of the second enzyme electrode is already reached, thus making it impossible to perform an accurate measurement of sucrose (in Comparative Example 1) and maltose (in Comparative Example 2).

Example 3

For the first enzyme electrode 2 in Fig. 1, a glucose detecting electrode having glucose oxidase immobilized thereon was employed, and for the second enzyme electrode 4 in Fig. 1, a glucose and lactose detecting electrode having glucose oxidase and $\beta$-galactosidase (Aspergillus oryzae, Sigma) immobilized thereon was employed.

The contruction of the first enzyme electrode 2 was the same as that in Example 1, and the second enzyme electrode 4 was prepared in the same manner as the second electrode 4 in Example 1. The other arrangement was the same as in Example 1.

In this system, 5 µl of aqueous solutions of glucose only (within a concentration range of 5 to 60 mM) were first injected through the injector 1, and a calibration curve as shown by line ℓ15 in Fig. 13 was obtained from the first enzyme electrode 2. Aqueous solutions of glucose only (within a concentration range of 5 to 70 mM) was injected in an amount of 5 µl through the injection means 1, and a calibration curve, as shown by line ℓ16 in Fig. 14, was obtained from the second enzyme electrode 4. By virtue of the function of the dilution pipeline 5, the output of the second electrode 4 remained within the linear range.

Aqueous solutions of lactose only (within a concentration range of 0 to 70 mM) were injected in an amount of 5 µl through the injection means 1, and a calibration curve shown by line ℓ17 in Fig. 15 was obtained from the second enzyme electrode 4.

Comparative Example 3

The measurements were carried out in the same manner as in Example 3 without using the dilution pipeline 5 in Fig. 1. It was confirmed that, as line ℓ18 in Fig. 16 shows, the calibration curve of the second enzyme electrode 4 showed a tendency for saturation at about 50 mM. Therefore, where samples contained glucose of 50 mM and lactose, the output of the second enzyme electrode 4 deviated from the measurable proportional range thereof, and hence it was found impossible to perform an accurate measurement of the lactose concentration.

Example 3 and Comparative Example 3 may be summarized as follows. In Example 3, the glucose concentration can be measured on the basis of the output current values of the first enzyme electrode 2 as shown in Fig. 13. It is also apparent from Figs. 14 and 15 that with samples containing glucose of 50 mM, the lactose concentration can be measured from the output current values of the first and second enzyme electrodes 2, 4. In contrast, it is confirmed that in Comparative Example 3, if the samples contain glucose of 50 mM, the upper limit of the proportional measuring range of the second enzyme electrode 4 is already reached as shown in Fig. 16, it being thus impossible to perform an accurate measurement of lactose.

**Claims**

**1.** Flow injection method for the quantitative determination of a substance (second substance) which is capable of being enzymatically converted into another substance (first substance), contained in a liquid sample by means of two enzyme electrodes (2,4) which detect the first substance, the second enzyme electrode (4) catalyzing the conversion of the second substance into the first substance prior to its detection, comprising the following steps:

(I) Determining the response ratio (k) of the two enzyme electrodes (2,4) regarding the first substance

by

(A) supplying a constant flow of a buffer solution,

(B) injecting the first substance into the flow of the buffer solution,

(C) passing the solution obtained in step (B) through a first measuring cell (3) comprising the first enzyme electrode (2) for detecting the first substance and measuring the output current ($I_a$)

(D) passing the solution after step (C) through a dilution pipeline (5;5a), and

(E) passing the solution after step (D) through a second measuring cell (11) comprising the second enzyme electrode (4) for detecting the first substance, and measuring the output current ($I_b$),

(II) calculating the response ratio k according to the equation α,

$$k = I_b/I_a \qquad (\alpha),$$

(III) selecting a suitable length of the dilution pipeline (5;5a) according to the concentration ratio of the first and the second substance,

(IV) determining the second substance

by

(I) injecting the sample to be analyzed containing the first substance and the second substance into the flow of the buffer solution,

(G) passing the solution obtained in step (I) through the first measuring cell (3) comprising the first enzyme electrode (2) and measuring output current ($I_1$),

(H) passing the solution after step (G) through the dilution pipeline (5;5a),

(J) passing the solution after step (H) through the second measuring cell (11) comprising the second enzyme electrode (4) adapted to convert the second substance to be determined into the first substance, and measuring the output current ($I_2$),

and

(V) calculating the current value $I_3$ corresponding to the amount of the second substance to be determined according to the equation ($\beta$),

$$I_3 = I_2 - k \cdot I_1 \qquad (\beta),$$

wherein k is the response ratio of the two enzyme electrodes determined in step (II).

**2.** The method according to claim 1,
characterized in that

- where the first substance is glucose, and the second substance is sucrose, a first enzyme electrode (2) having glucose oxidase immobilized thereon, and a second enzyme electrode (4) having glucose oxidase, mutarotase and invertase immobilized thereon are used,
- where the first substance is glucose and the second substance is maltose, a first enzyme electrode (2) having glucose oxidase immobilized thereon, and a second enzyme electrode (4) having glucose oxidase, mutarotase and $\alpha$-glucosidase immobilized thereon are used,
- where the first substance is glucose, and the second substance is a $\beta$-glucoside, a first enzyme electrode (2) having glucose oxidase immobilized thereon, and a second enzyme electrode (4) having glucose oxidase and $\beta$-glucosidase immobilized thereon are used,
- where the first substance is glucose, and the second substances are maltooligosugars, a first enzyme electrode (2) having glucose oxidase immobilized thereon, and a second enzyme electrode (4) having glucose oxidase and glucoamylase immobilized thereon are used,
- where the first substance is glucose, and the second substance is lactose, a first enzyme electrode (2) having glucose oxidase immobilized thereon, and a second enzyme electrode (4) having glucose oxidase and lactase immobilized thereon are used,

and,

- where the first substance is free cholesterol, and the second substance is a cholesteryl ester, a first enzyme electrode (2) having cholesterol oxidase immobilized thereon, and a second enzyme electrode (4) having cholesterol oxidase and cholesterol esterase immobilized thereon are used.

**3.** The method according to claim 1 or 2,
characterized in that also the first substance is quantitatively determined from the output current ($I_1$) of the first measuring cell measured in step (G) and corresponding to the amount of the first substance.

**4.** The method according to one of claims 1 to 3,
characterized in that steps (I) (B) to (V) are carried out intermittently.

**5.** Flow injection apparatus for the quantitative determination of a substance (second substance) which is capable of being enzymatically converted into another substance (first substance), contained in a liquid sample by means of two enzyme electrodes (2,4), particularly for carrying out the method according to claims 1 to 4,
comprising

- supply means (6) for continuously supplying a buffer solution (8) at a predetermined flow rate,
- injection means (1) for injecting samples to be measured into the flow of the buffer solution (8),
- a first measuring cell (3) comprising a first enzyme electrode (2) for detecting the first substance and being provided downstream of the injecting means (1),
- a dilution pipeline (5,5a) provided downstream of the first measuring cell (3) for diluting the sample along its flow direction,
- a second measuring cell (11) provided at the downstream end of the dilution pipeline (5) comprising a second enzyme electrode (4) having an enzyme immobilized thereon for catalyzing the conversion of the second substance into the first substance and for detecting (a) the first substance originally contained in the sample and (b) the first substance resulting from the conversion of the second substance,
- means (16) for calculating the response ratio k according to the equation $\alpha$,

$$k = I_b/I_a \quad (\alpha),$$

wherein
$I_a$ is the measured output current value of the first measuring cell (3),
and
$I_b$ is the measured output current value of the second measuring cell (11),
and for calculating the current value $I_3$ corresponding to the concentration of the second substance to be determined according to the equation $\beta$,

$$I_3 = I_2 - k \bullet I_1 \quad (\beta),$$

wherein
$I_1$ is the output current of the first measuring cell (3), and
$I_2$ is the output current of the second measuring cell (11),
and
- means (14,15) for selecting a suitable length of the dilution pipeline (5;5a) according to the concentration ratio of the first and the second substance,

the apparatus being adapted to carry out the following steps:
(I) Determining the response ratio (k) of the two enzyme electrodes (2, 4) regarding the first substance
by
(A) supplying a constant flow of a buffer solution (8),
(B) injecting the first substance into the flow of the buffer solution,
(C) passing the solution obtained in step (B) through the first measuring cell (3), and measuring the output current ($I_a$),
(D) passing the solution after step (C) through the dilution pipeline (5;5a), and
(E) passing the solution after step (D) through the second measuring cell (11) and measuring the output current ($I_b$),
(II) calculating the response ratio k according to the equation $\alpha$,

$$k = I_b/I_a \quad (\alpha),$$

(III) selecting a suitable length of the dilution pipeline (5;5a) according to the concentration ratio of the first and the second substance,
(IV) determining the second substance
by
(I) injecting the sample to be analyzed containing the first substance and the second substance into the flow of the buffer solution,
(G) passing the solution obtained in step (I) through the first measuring cell (3) and measuring the output current ($I_1$),
(H) passing the solution after step (G) through the dilution pipeline (5;5a), and
(J) passing the solution after step (H) through the second measuring cell (11) and measuring the output current ($I_2$),
and
(V) calculating the current value $I_3$ corresponding to the amount of the second substance to be determined according to the equation $\beta$

$$I_3 = I_2 - k \bullet I_1 \quad (\beta).$$

**6.** The apparatus according to claim 5, characterized by one or more of the following features:
- The injection means (1) are a HPLC injector;
- the dilution pipeline (5;5a) has an inner diameter of the flow path of 0,1 to 2,0 mm and an axial length of 0,2 to 100 m;
- the dilution pipeline (5;5a) is made of a synthetic resin;
- the dilution pipeline (5;5a) is made of stainless steel;
- the dilution pipeline (5;5a) is coiled;
- it comprises a plurality of dilution pipelines (5, 5a), each having a different inner diameter and/or a different axial length, respectively, and switching valves (14, 15) are provided to selectively

connect one of the dilution pipelines (5, 5a) between the first measuring cell (3) and the second measuring cell (11).

**7.** The apparatus according to claim 5 or 6,
characterized in that

- where the first substance is glucose, and the second substance is sucrose, the first enzyme electrode (2) has glucose oxidase immobilized thereon, and the second enzyme electrode (4) has glucose oxidase, mutarotase and invertase immobilized thereon;
- where the first substance is glucose, and the second substance is maltose, the first enzyme electrode (2) has glucose oxidase immobilized thereon, and the second enzyme electrode (4) has glucose oxidase, mutarotase and $\alpha$ -glucosidase immobilized thereon;
- where the first substance is glucose, and the second substance is a $\beta$-glucoside, the first enzyme electrode (2) has glucose oxidase immobilized thereon, and the second enzyme electrode (4) has glucose oxidase and $\beta$-glucosidase immobilized thereon;
- where the first substance is glucose, and the second substances are maltooligosugars, the first enzyme electrode (2) has glucose oxidase immobilized thereon, and the second enzyme electrode (4) has glucose oxidase and glucoamylase immobilized thereon;
- where the first substance is glucose, and the second substance is lactose, the first enzyme electrode (2) has glucose oxidase immobilized thereon, and the second enzyme electrode (4) has glucose oxidase and lactase immobilized thereon,
  and,
- where the first substance is free cholesterol, and the second substance is a cholesteryl ester, the first enzyme electrode (2) has cholesterol oxidase immobilized thereon, and the second enzyme electrode (4) has cholesterol oxidase and cholesterol esterase immobilized thereon.

**8.** The apparatus according to one of claims 5 to 7,
characterized in that the calculating means (16) are adapted to quantitatively determine also the first substance from the output current ($I_1$) of the first measuring cell (2) measured in step (G) and corresponding to the amount of the first substance.

**9.** The apparatus according to one of claims 5 to 8,
characterized by
means for intermittently carrying out the steps (I)(B) to (V).

## Patentansprüche

**1.** Flow-injection-Verfahren zur quantitativen Bestimmung einer in einer flüssigen Probe enthaltenen Substanz (zweite Substanz), die enzymatisch in eine andere Substanz (erste Substanz) umgewandelt werden kann, mit zwei Enzymelektroden (2, 4), welche die erste Substanz erfassen, wobei die zweite Enzymelektrode (4) die Umwandlung der zweiten Substanz in die erste Substanz vor ihrer Erfassung katalysiert,
das folgende Schritte umfaßt:

(I) Bestimmung des Ansprechverhältnisses (k) der beiden Enzymelektroden (2, 4) bezüglich der ersten Substanz
durch

(A) Vorsehen eines konstanten Stroms einer Pufferlösung
(B) Einspritzen der ersten Substanz in den Strom der Pufferlösung,
(C) Hindurchleiten der in Schritt (B) erhaltenen Lösung durch eine erste Meßzelle (3), welche die erste Enzymelektrode (2) enthält, zur Erfassung der ersten Substanz und Messung des Ausgangsstroms ($I_a$),
(D) Hindurchleiten der Lösung nach Schritt (C) durch eine Verdünnungspipeline (5; 5a), und
(E) Hindurchleiten der Lösung nach Schritt (D) durch eine zweite Meßzelle (11), welche die zweite Enzymelektrode (4) enthält, zur Erfassung der ersten Substanz und Messung des Ausgangsstroms ($I_b$),

(II) Berechnung des Ansprechverhältnisses k nach der Gleichung $\alpha$,

$$k = I_b/I_a \qquad (\alpha),$$

(III) Wahl einer geeigneten Länge der Verdünnungspipeline (5; 5a) in Abhängigkeit vom Konzentrationsverhältnis der ersten und der zweiten Substanz,
(IV) Bestimmung der zweiten Substanz
durch
(F) Einspritzen der zu analysierenden Probe, welche die erste und die zweite Substanz enthält, in den Strom der Pufferlösung,
(G) Hindurchleiten der in Schritt (F) erhaltenen Lösung durch die erste Meßzelle (3), welche die erste Enzymelektrode (2) enthält, und Messung des Ausgangsstroms ($I_1$),
(H) Hindurchleiten der Lösung nach Schritt (G) durch die Verdünnungspipeline (5; 5a) und
(J) Hindurchleiten der Lösung nach Schritt (H) durch die zweite Meßzelle (11), welche die zweite Enzymelektrode (4) enthält, welche die zweite, zu bestimmende Substanz in die erste Substanz umzuwandeln vermag, und Messung des Ausgangsstroms ($I_2$),
sowie
(V) Berechnung des der Menge der zweiten, zu bestimmenden Substanz entsprechenden Wertes des Stroms $I_3$ nach der Gleichung ($\beta$),

$$I_3 = I_2 - k \cdot I_1 \quad (\beta),$$

worin k das in Schritt (II) bestimmte Ansprechverhältnis bedeutet.

**2.** Verfahren nach Anspruch 1,
gekennzeichnet durch
- Verwendung einer ersten Enzymelektrode (2), die darauf immobilisierte Glucoseoxidase aufweist, und einer zweiten Enzymelektrode (4), die darauf immobilisierte Glucoseoxidase, Mutarotase und Invertase aufweist, wenn die erste Substanz Glucose und die zweite Substanz Saccharose sind,
- Verwendung einer ersten Enzymelektrode (2), die darauf immobilisierte Glucoseoxidase aufweist, und einer zweiten Enzymelektrode (4), die darauf immobilisierte Glucoseoxidase, Mutarotase und $\alpha$-Glucosidase aufweist, wenn die erste Substanz Glucose und die zweite Substanz Maltose sind,
- Verwendung einer ersten Enzymelektrode (2), die darauf immobilisierte Glucoseoxidase aufweist, und einer zweiten Enzymelektrode (4), die darauf immobilisierte Glucoseoxidase und $\beta$-Glucosidase aufweist,
wenn die erste Substanz Glucose und die zweite Substanz ein $\beta$-Glucosid sind,
- Verwendung einer ersten Enzymelektrode (2), die darauf immobiliserte Glucoseoxidase aufweist, und einer zweiten Enzymelektrode (4), die darauf immobilisierte Glucoseoxidase und Glucoamylase aufweist,
wenn die erste Substanz Glucose und die zweiten Substanzen Oligomaltosen sind,
- Verwendung einer ersten Enzymelektrode (2), die darauf immobilisierte Glucoseoxidase aufweist und einer zweiten Enzymelektrode (4), die darauf immobilisierte Glucoseoxidase und Lactase aufweist,
wenn die erste Substanz Glucose und die zweite Substanz Lactose sind, und
- Verwendung einer ersten Enzymelektrode (2), die darauf immobilisierte Cholesterinoxidase aufweist, und einer zweiten Enzymelektrode (4), die darauf immobilisierte Cholesterinoxidase und Cholesterinesterase aufweist,
wenn die erste Substanz freies Cholesterin und die zweite Substanz ein Cholesterinester sind.

**3.** Verfahren nach Anspruch 1 oder 2,
dadurch gekennzeichnet, daß auch die erste Substanz aus dem Ausgangsstrom ($I_1$) der ersten Meßzelle quantitativ bestimmt wird, der in Schritt (G) gemessen wurde und der Menge der ersten Substanz entspricht.

**4.** Verfahren nach einem der Ansprüche 1 bis 3,
dadurch gekennzeichnet, daß die Schritte (I), (B) bis (V) intermittierend durchgeführt werden.

**5.** Flow-injection-Vorrichtung zur quantitativen Bestimmung einer in einer flüssigen Probe enthaltenen Substanz (zweite Substanz), die enzymatisch in eine andere Substanz (erste Substanz) umgewandelt werden kann,
mit zwei Enzymelektroden (2, 4),
insbesondere zur Durchführung des Verfahrens nach den Ansprüchen 1 bis 4,

die aufweist:

- eine Fördereinrichtung (6) zur kontinuierlichen Förderung einer Pufferlösung (8) mit vorgegebenem Durchsatz,
- eine Einspritzeinrichtung (1) zum Einspritzen der zu messenden Proben in den Strom der Pufferlösung (8),
- eine erste Meßzelle (3), die eine erste Enzymelektrode (2) zur Erfassung der ersten Substanz aufweist und stromab von der Einspritzeinrichtung (1) vorgesehen ist,
- eine Verdünnungspipeline (5; 5a), die stromab von der ersten Meßzelle (3) vorgesehen ist und zur Verdünnung der Probe längs ihrer Strömungsrichtung dient,
- eine zweite Meßzelle (11), die am stromab liegendenden Ende der Verdünnungspipeline (5) vorgesehen ist und eine zweite Enzymelektrode (4) aufweist, die ein darauf immobilisiertes Enzym aufweist, das die Umwandlung der zweiten Substanz in die erste Substanz katalysiert und zur Erfassung (a) der ursprünglich in der Probe enthaltenen ersten Substanz und (b) der aus der Umwandlung der zweiten Substanz resultierenden ersten Substanz dient,
- eine Einrichtung (16) zur Berechnung des Ansprechverhältnisses k nach der Gleichung $\alpha$,

  $$k = I_b/I_a \qquad (\alpha);$$

  worin
  $I_a$ den gemessenen Wert des Ausgangsstroms der ersten Meßzelle (3),
  und
  $I_b$ den gemessenen Wert des Ausgangsstroms der zweiten Meßzelle (11)
  bedeuten,
  und zur Berechnung des der Konzentration der zweiten, zu bestimmenden Substanz entsprechenden Wertes des Stroms $I_3$ nach der Gleichung $\beta$,

  $$I_3 = I_2 - k \cdot I_1 \qquad (\beta),$$

  worin
  $I_1$ den Ausgansstrom der ersten Meßzelle (3) und
  $I_2$ den Ausgangsstrom der zweiten Meßzelle (11) bedeuten,
  und
- eine Einrichtung (14, 15) zur Wahl einer geeigneten Länge der Verdünnungspipeline (5; 5a) in Abhängigkeit vom Konzentrationsverhältnis der ersten und der zweiten Substanz,

wobei die Vorrichtung so ausgebildet ist, daß sie folgende Schritte durchführen kann:

(I) Bestimmung des Ansprechverhältnisses (k) der beiden Enzymelektroden (2, 4) bezüglich der ersten Substanz
durch

(A) Vorsehen eines konstanten Stroms einer Pufferlösung (8),
(B) Einspritzen der ersten Substanz in den Strom der Pufferlösung,
(C) Hindurchleiten der in Schritt (B) erhaltenen Lösung durch die erste Meßzelle (3) und Messung des Ausgangsstroms ($I_a$),
(D) Hindurchleiten der Lösung nach Schritt (C) durch die Verdünnungspipeline (5; 5a), und
(E) Hindurchleiten der Lösung nach Schritt (D) durch die zweite Meßzelle (11), und Messung des Ausgangsstroms ($I_b$),

(II) Berechnung des Ansprechverhältnisses k nach der Gleichung $\alpha$,

$$k = I_b/I_a \qquad (\alpha),$$

(III) Wahl einer geeigneten Länge der Verdünnungspipeline (5; 5a) in Abhängigkeit vom Konzentrationsverhältnis der ersten und der zweiten Substanz,
(IV) Bestimmung der zweiten Substanz
durch

(F) Einspritzen der zu analysierenden Probe, welche die erste und die zweite Substanz enthält, in den Strom der Pufferlösung,
(G) Hindurchleiten der in Schritt (F) erhaltenen Lösung durch die erste Meßzelle (3) und Messung des Ausgangsstroms ($I_1$),
(H) Hindurchleiten der Lösung nach Schritt (G) durch die Verdünnungspipeline (5; 5a), und

(J) Hindurchleiten der Lösung nach Schritt (H) durch die zweite Meßzelle (11), und Messung des Ausgangsstroms ($I_2$),
und

(V) Berechnung des der Menge der zweiten, zu bestimmenden Substanz entsprechenden Wertes des Stroms $I_3$ nach der Gleichung ($\beta$),

$$I_3 = I_2 - k \cdot I_1 \qquad (\beta).$$

**6.** Vorrichtung nach Anspruch 5, gekennzeichnet durch ein oder mehrere der folgenden Merkmale:
- Die Einspritzeinrichtung (1) ist eine HPLC-Einspritzeinrichtung;
- die Verdünnungspipeline (5; 5a) weist einen Strömungskanal mit einem Innendurchmesser von 0,1 bis 2,0 mm und einer axialen Länge von 0,2 bis 100 m auf;
- die Verdünnungspipeline (5; 5a) besteht aus einem Kunstharz;
- die Verdünnungspipeline (5; 5a) besteht aus rostfreiem Stahl;
- die Verdünnungspipeline (5; 5a) liegt in einer gewickelten Form vor;
- es sind mehrere Verdünnungspipelines (5; 5a), die jeweils unterschiedliche Innendurchmesser und/oder unterschiedliche axiale Länge aufweisen, sowie Schaltventile (14, 15) vorgesehen, mit denen eine der Verdünnungspipelines (5, 5a) zwischen die erste Meßzelle (3) und die zweite Meßzelle (11) eingeschaltet werden kann.

**7.** Vorrichtung nach Anspruch 5 oder 6,
gekennzeichnet durch
- eine erste Enzymelektrode (2), die darauf immobilisierte Glucoseoxidase aufweist, und eine zweite Enzymelektrode (4), die darauf immobilisierte Glucoseoxidase, Mutarotase und Invertase aufweist,
  wenn die erste Substanz Glucose und die zweite Substanz saccharose sind;
- eine erste Enzymelektrode (2), die darauf immobilisierte Glucoseoxidase aufweist, und eine zweite Enzymelektrode (4), die darauf immobilisierte Glucoseoxidase, Mutarotase und $\alpha$-Glucosidase aufweist,
  wenn die erste Substanz Glucose und die zweite Substanz Maltose sind,
- eine erste Enzymelektrode (2), die darauf immobilisierte Glucoseoxidase aufweist, und eine zweite Enzymelektrode (4), die darauf immobilisierte Glucoseoxidase und $\beta$-Glucosidase aufweist,
  wenn die erste Substanz Glucose und die zweite Substanz ein $\beta$-Glucosid sind,
- eine erste Enzymelektrode (2), die darauf immobiliserte Glucoseoxidase aufweist, und eine zweite Enzymelektrode (4), die darauf immobilisierte Glucoseoxidase und Glucoamylase aufweist,
  wenn die erste Substanz Glucose und die zweiten Substanzen Oligomaltosen sind,
- eine erste Enzymelektrode (2), die darauf immobilisierte Glucoseoxidase aufweist und eine zweite Enzymelektrode (4), die darauf immobilisierte Glucoseoxidase und Lactase aufweist,
  wenn die erste Substanz Glucose und die zweite Substanz Lactose sind, und
- eine erste Enzymelektrode (2), die darauf immobilisierte Cholesterinoxidase aufweist, und eine zweite

Enzymelektrode (4), die darauf immobilisierte Cholesterinoxidase und Cholesterinesterase aufweist,
wenn die erste Substanz freies Cholesterin und die zweite Substanz ein Cholesterinester sind.

**8.** Vorrichtung nach einem der Ansprüche 5 bis 7,
dadurch gekennzeichnet, daß die Einrichtung (16) zur Berechnung des Ansprechverhältnisses so ausgebildet ist, daß sie auch die erste Substanz aus dem Ausgangsstrom ($I_1$) der ersten Meßzelle (2) quantitativ bestimmt, der in Schritt (G) gemessen wurde und der Menge der ersten Substanz entspricht.

**9.** Vorrichtung nach einem der Ansprüche 5 bis 8,
gekennzeichnet durch eine Einrichtung zur intermittierenden Durchführung der Schritte (I) (B) bis (V).

## Revendications

**1.** Procédé d'injection dans un courant, destiné à la détermination quantitative d'une substance (seconde substance) susceptible d'être convertie enzymatiquement en une autre substance (première substance), contenue dans un échantillon liquide, au moyen de deux électrodes enzymatiques (2,4) qui détectent la première substance, la seconde électrode enzymatique (4) catalysant la conversion de la

seconde substance en première substance avant sa détection, comprenant les étapes suivantes consistant à :

(I) déterminer le rapport de réponse (k)des deux électrodes enzymatiques (2,4) concernant la première substance par

(A) apport d'un courant constant d'une solution tampon,

(B) injection de la première substance dans le courant de solution tampon,

(C) passage de la solution obtenue à l'étape (B) par une première cellule de mesure (3) comprenant la première électrode enzymatique (2) en vue de la détection de la première substance et mesure du courant de sortie ($I_a$),

(D) passage de la solution après l'étape (C) par une canalisation de dilution (5;5a), et

(E)passage de la solution après l'étape (D) par une seconde cellule de mesure (11) comprenant la seconde électrode enzymatique (4) en vue de la détection de la première substance et mesure du courant de sortie ($I_b$),

(II) calculer le rapport de réponse (k)selon l'équation $\alpha$,

$$k = I_b/I_a \qquad (\alpha),$$

(III) sélectionner une longueur appropriée pour la canalisation de dilution (5;5a) selon le rapport de concentration de la première et de la seconde substances,

(IV) déterminer la seconde substance par

(F) injection de l'échantillon à analyser contenant la première substance et la seconde substance dans le courant de solution tampon,

(G) passage de la solution obtenue à l'étape (F) par la première cellule de mesure (3) comprenant la première électrode enzymatique (2) et mesure du courant de sortie ($I_1$),

(H) passage de la solution après l'étape (G) par la canalisation de dilution (5;5a),

(J) passage de la solution après l'étape (H) par la seconde cellule de mesure (11) comprenant la seconde électrode enzymatique (4) conçue pour convertir la seconde substance à déterminer en première substance et mesure du courant de sortie ($I_2$),

et

(V)calculer la valeur ($I_3$) du courant correspondant à la quantité de seconde substance à déterminer selon l'équation ($\beta$),

$$I_3 = I_2 - k \bullet I_1 \qquad (\beta)$$

dans laquelle k est le rapport de réponse des deux électrodes enzymatiques déterminé à l'étape (II).

**2.** Procédé selon la revendication 1, caractérisé en ce que :

- lorsque la première substance est du glucose et que la seconde substance est du saccharose, on utilise une première électrode enzymatique (2) sur laquelle est immobilisée de la glucose-oxydase et une seconde électrode enzymatique (4) sur laquelle sont immobilisées de la glucose-oxydase, de la mutarotase et de l'invertase,
- lorsque la première substance est du glucose et que la seconde substance est du maltose, on utilise une première électrode enzymatique (2) sur laquelle est immobilisée de la glucose-oxydase et une seconde électrode enzymatique (4) sur laquelle sont immobilisées de la glucose-oxydase, de la mutarotase et de l'$\alpha$-glucosidase,
- lorsque la première substance est du glucose et que la seconde substance est un $\beta$-glucoside, on utilise une première électrode enzymatique (2) sur laquelle est immobilisée de la glucose-oxydase et une seconde électrode enzymatique (4) sur laquelle sont immobilisées de la glucose-oxydase et de la $\beta$-glucosidase,
- lorsque la première substance est du glucose et que les secondes substances sont des maltooligosucres, on utilise une première électrode enzymatique (2) sur laquelle est immobilisée de la glucose-oxydase et une seconde électrode enzymatique (4) sur laquelle sont immobilisées de la glucose-oxydase et de la glucoamylase,
- lorsque la première substance est du glucose et que la seconde substance est du lactose, on utilise une première électrode enzymatique (2) sur laquelle est immobilisée de la glucose-oxydase et une seconde électrode enzymatique (4) sur laquelle sont immobilisées de la glucose-oxydase et de la lactase,

et

- lorsque la première substance est du cholestérol libre et que la seconde substance est un ester de cholestéryle, on utilise une première électrode enzymatique (2) sur laquelle est immobilisée de la cholestérol-oxydase et une seconde électrode enzymatique (4) sur laquelle sont immobilisées de la cholestérol-oxydase et de la cholestérol-estérase.

**3.** Procédé selon la revendication 1 ou 2, caractérisé en ce que la première substance est également déterminée quantitativement d'après le courant de sortie ($I_1$) de la première cellule de mesure mesuré à l'étape (G) et correspondant à la quantité de première substance.

**4.** Procédé selon l'une des revendications 1 à 3, caractérisé en ce que les étapes (I)(B) à (V) sont effectuées de façon intermittente.

**5.** Appareil d'injection dans un courant, destiné à la détermination quantitative d'une substance (seconde substance) susceptible d'être convertie enzymatiquement en une autre substance (première substance), contenue dans un échantillon liquide, au moyen de deux électrodes enzymatiques (2,4), destiné plus particulièrement à mettre en oeuvre le procédé selon les revendications 1 à 4, comprenant :

- un moyen d'apport (6) destiné à apporter continûment une solution tampon (8) à un débit prédéterminé,
- un moyen d'injection (1) destiné à injecter des échantillons à mesurer dans le courant de solution tampon (8),
- une première cellule de mesure (3) comprenant une première électrode enzymatique (2) destinée à détecter la première substance et disposée en aval du moyen d'injection (1),
- une canalisation de dilution (5; 5a) disposée en aval de la première cellule de mesure (3) pour diluer l'échantillon dans la direction de son écoulement,
- une seconde cellule de mesure (11) disposée à l'extrémité aval de la canalisation de dilution (5), comprenant une seconde électrode enzymatique (4) sur laquelle est immobilisée une enzyme pour catalyser la conversion de la seconde substance en première substance et pour détecter (a) la première substance contenue à l'origine dans l'échantillon et (b) la première substance résultant de la conversion de la seconde substance,
- un moyen (16) de calcul du rapport de réponse k selon l'équation $\alpha$,

$$k = I_b/I_a \qquad (\alpha)$$

dans laquelle
$I_a$ est la valeur mesurée pour le courant de sortie de la première cellule de mesure (3) et
$I_b$ est la valeur mesurée pour le courant de sortie de la seconde cellule de mesure (11),
et de calcul de la valeur $I_3$ du courant correspondant à la concentration de la seconde substance à déterminer selon l'équation $\beta$,

$$I_3 = I_2 - k \bullet I_1 \qquad (\beta)$$

dans laquelle
$I_1$ est le courant de sortie de la première cellule de mesure (3) et
$I_2$ est le courant de sortie de la seconde cellule de mesure (11),
et

- des moyens (14, 15) de sélection d'une longueur appropriée pour la canalisation de dilution (5;5a) selon le rapport de concentration de la première et de la seconde substances,

l'appareil étant conçu pour effectuer les étapes suivantes, consistant à :

(I) déterminer le rapport de réponse (k) des deux électrodes enzymatiques (2,4) concernant la première substance par

(A) apport d'un courant constant d'une solution tampon (8),
(B) injection de la première substance dans le courant de solution tampon,
(C) passage de la solution obtenue à l'étape (B) par la première cellule de mesure (3) et mesure du courant de sortie ($I_a$),
(D) passage de la solution après l'étape (C) par la canalisation de dilution (5;5a) et
(E) passage de la solution après l'étape (D) par la seconde cellule de mesure (11) et mesure du courant de sortie ($I_b$),

(II) calculer le rapport de réponse selon l'équation $\alpha$,

$$k = I_b/I_a \qquad (\alpha)$$

(III) sélectionner une longueur appropriée pour la canalisation de dilution (5;5a) selon le rapport de concentration de la première et de la seconde substances,

(IV) déterminer la seconde substance par

(F) injection de l'échantillon à analyser contenant la première substance et la seconde substance dans le courant de solution tampon,

(G) passage de la solution obtenue à l'étape (F) par la première cellule de mesure (3) et mesure du courant de sortie ($I_1$),

(H) passage de la solution après l'étape (G) par la canalisation de dilution (5;5a), et

(J) passage de la solution après l'étape (H) par la seconde cellule de mesure (11) et mesure du courant de sortie ($I_2$),

et

(V) calculer la valeur ($I_3$) du courant correspondant à la quantité de seconde substance à déterminer selon l'équation ($\beta$),

$$I_3 = I_2 - k \bullet I_1 \qquad (\beta).$$

**6.** Appareil selon la revendication 5, caractérisé par une ou plusieurs des caractéristiques suivantes :

- le moyen d'injection (1) est un injecteur de HPLC;
- la canalisation de dilution (5;5a) présente une voie d'écoulement de diamètre interne compris entre 0,1 et 2,0 mm et une longueur axiale comprise entre 0,2 et 100m;
- la canalisation de dilution (5;5a) est en résine synthétique;
- la canalisation de dilution (5;5a) est en acier inoxydable;
- la canalisation de dilution (5;5a) est enroulée;
- l'appareil comprend une pluralité de canalisations de dilution (5;5a) dont chacune présente respectivement un diamètre interne différent et/ou une longueur axiale différente et des vannes de commutation (14, 15) sont prévues pour connecter sélectivement une des canalisations de dilution (5;5a) entre la première cellule de mesure (3) et la seconde cellule de mesure (11).

**7.** Appareil selon la revendication 5 ou 6, caractérisé en ce que :

- lorsque la première substance est du glucose et que la seconde substance est du saccharose, la première électrode enzymatique (2) porte de la glucose-oxydase immobilisée et la seconde électrode enzymatique (4) porte de la glucose-oxydase, de la mutarotase et de l'invertase immobilisées;
- lorsque la première substance est du glucose et que la seconde substance est du maltose, la première électrode enzymatique (2) porte de la glucose-oxydase immobilisée et la seconde électrode enzymatique (4) porte de la glucose-oxydase, de la mutarotase et de l'$\alpha$-glucosidase immobilisées;
- lorsque la première substance est du glucose et que la seconde substance est un $\beta$-glucoside, la première électrode enzymatique (2) porte de la glucose-oxydase immobilisée et la seconde électrode enzymatique (4) porte de la glucose-oxydase et de la $\beta$-glucosidase immobilisées;
- lorsque la première substance est du glucose et que les secondes substances sont des maltooligosucres, la première électrode enzymatique (2) porte de la glucose-oxydase immobilisée et la seconde électrode enzymatique (4) porte de la glucose-oxydase et de la glucoamylase immobilisées;
- lorsque la première substance est du glucose et que la seconde substance est du lactose, la première électrode enzymatique (2) porte de la glucose-oxydase immobilisée et la seconde électrode enzymatique (4) porte de la glucose-oxydase et de la lactase immobilisées,

  et
- lorsque la première substance est du cholestérol libre et que la seconde substance est un ester de cholestéryle, la première électrode enzymatique (2) porte de la cholestérol-oxydase immobilisée et la seconde électrode enzymatique (4) porte de la cholestérol-oxydase et de la cholestérol-estérase immobilisées.

**8.** Appareil selon l'une des revendications 5 à 7, caractérisé en ce que le moyen de calcul (16) est conçu pour déterminer également quantitativement la première substance d'après le courant de sortie ($I_1$) de la première cellule de mesure (2) mesuré à l'étape (G) et correspondant à la quantité de première substance.

**9.** Appareil selon l'une des revendications 5 à 8, caractérisé par des moyens permettant de mettre en oeuvre de façon intermittente les étapes (I)(B) à (V).

Fig. 1

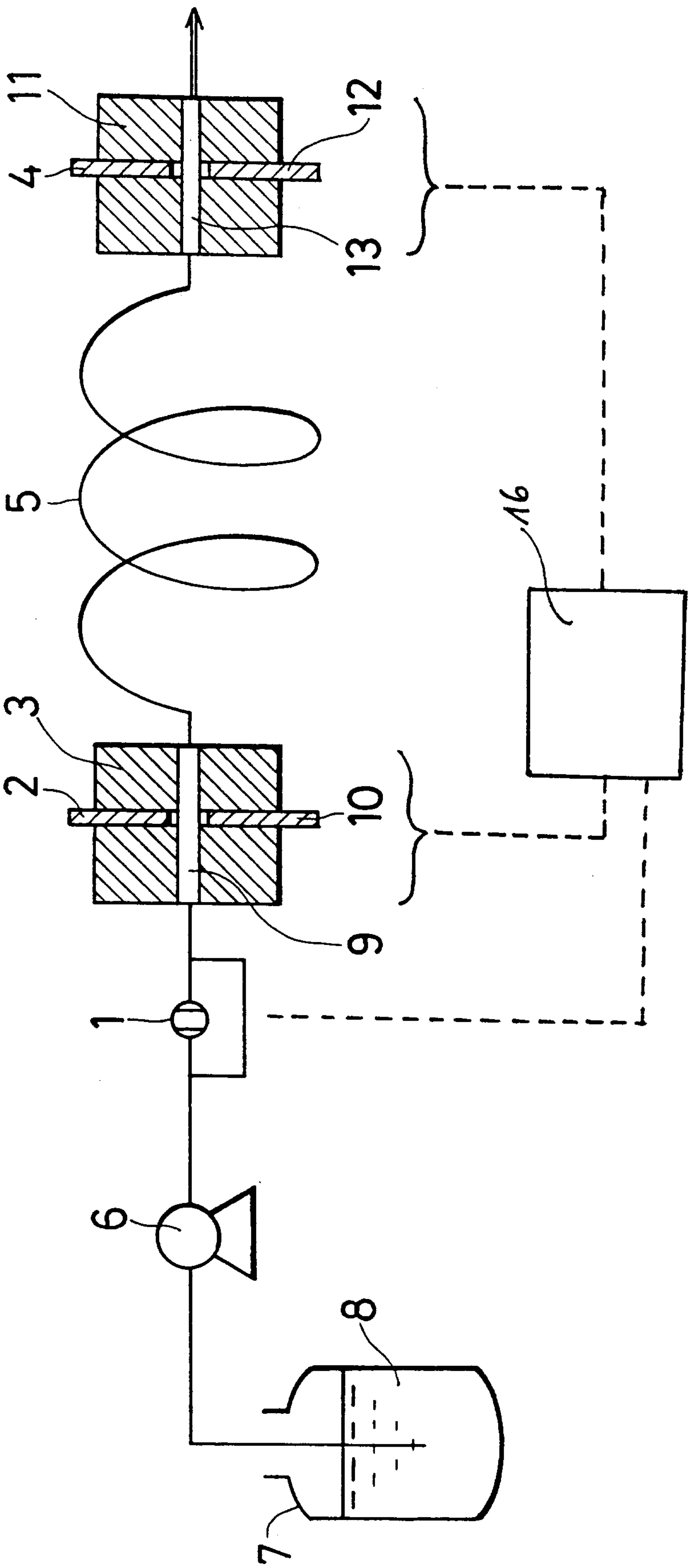

Fig.2

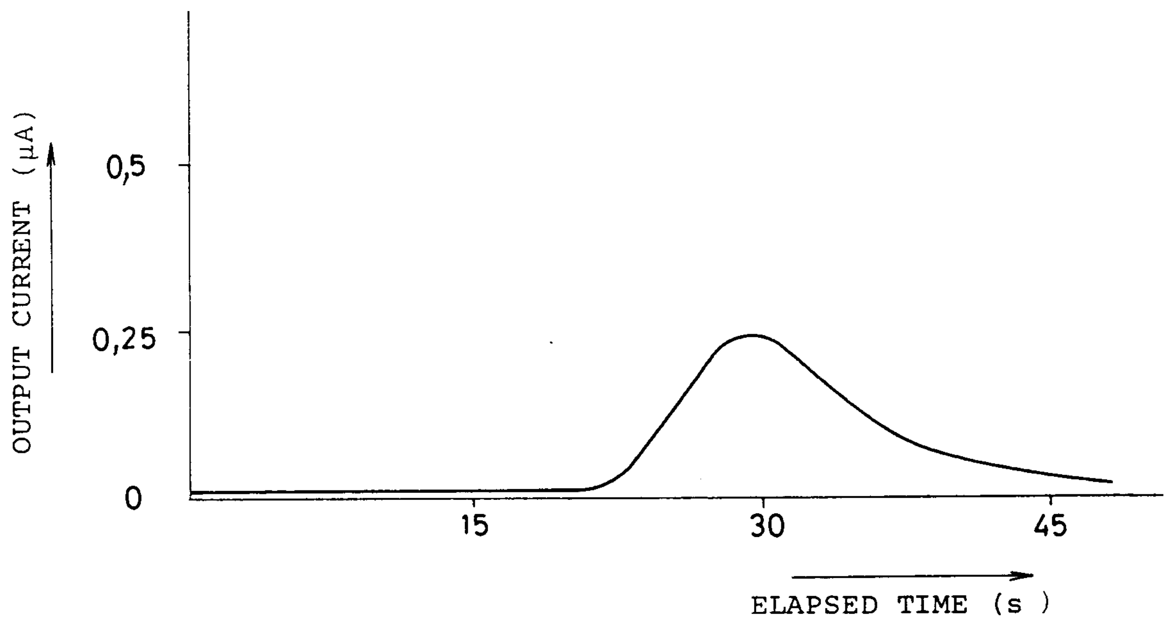
OUTPUT CURRENT (μA)
0,5
0,25
0
15
30
45
ELAPSED TIME (s)

Fig.3

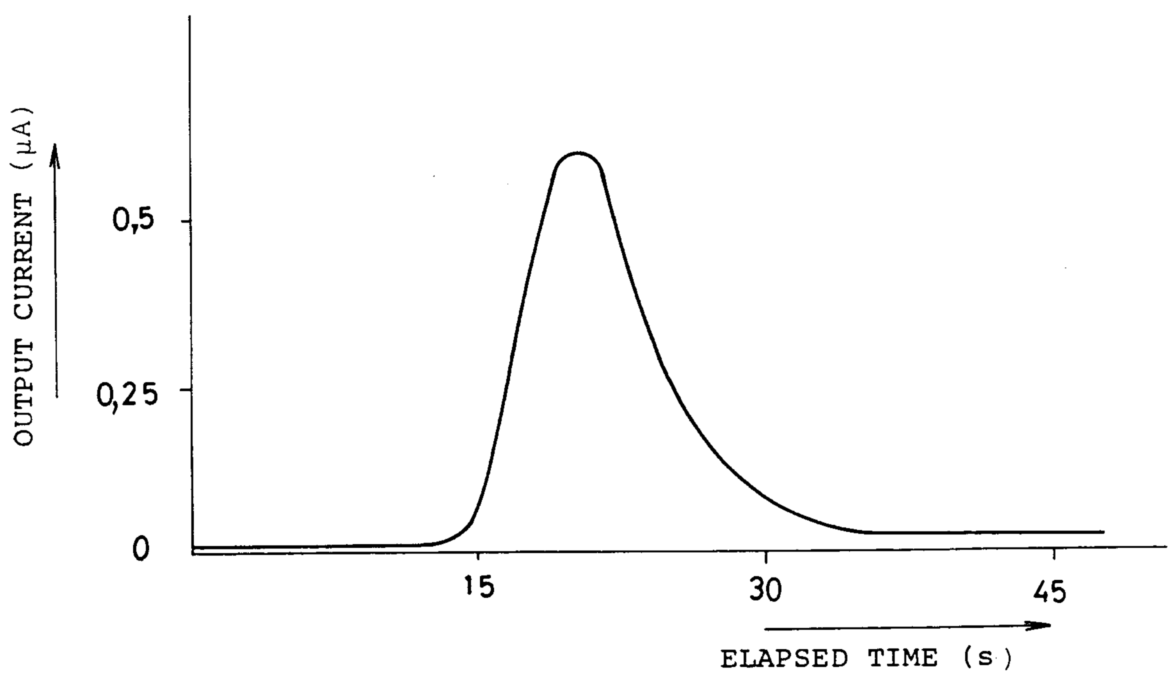
OUTPUT CURRENT (μA)
0,5
0,25
0
15
30
45
ELAPSED TIME (s)

Fig. 4

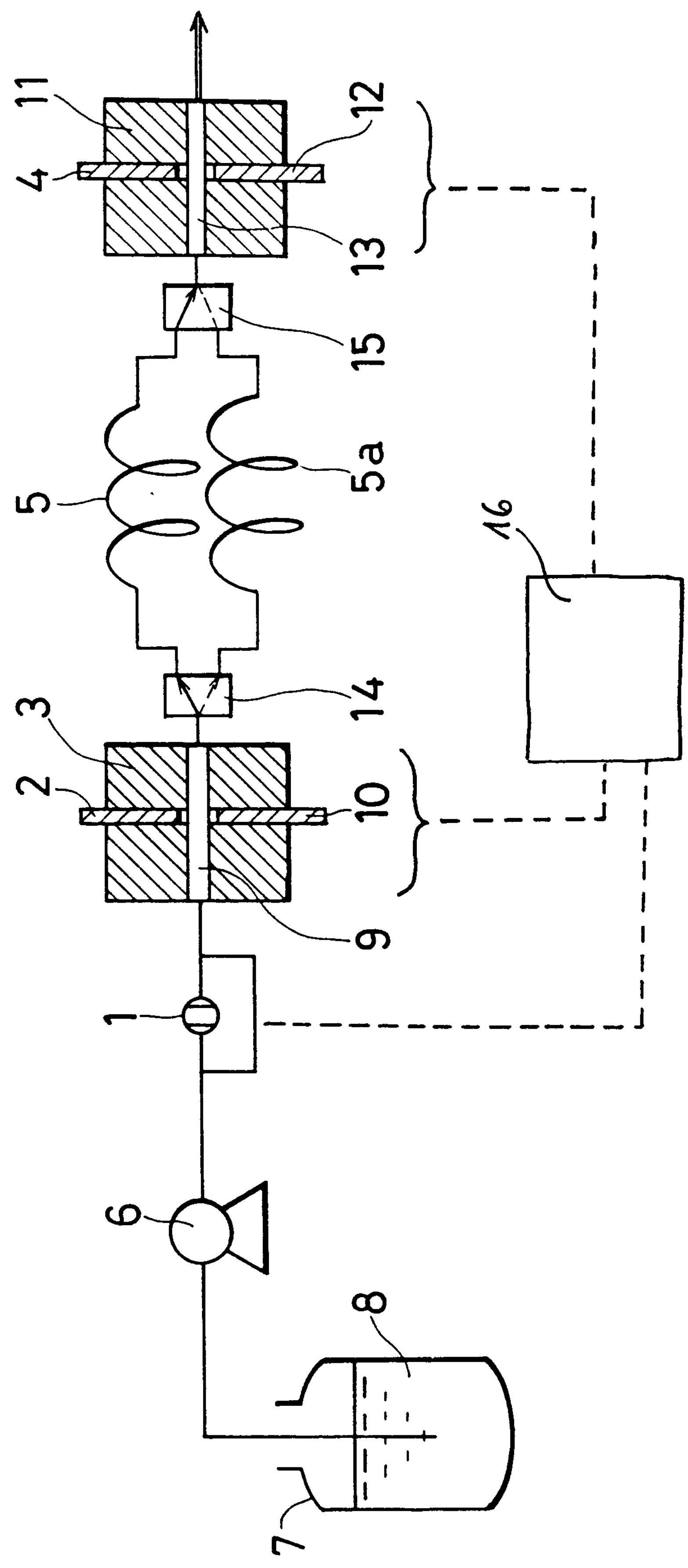

Fig.5

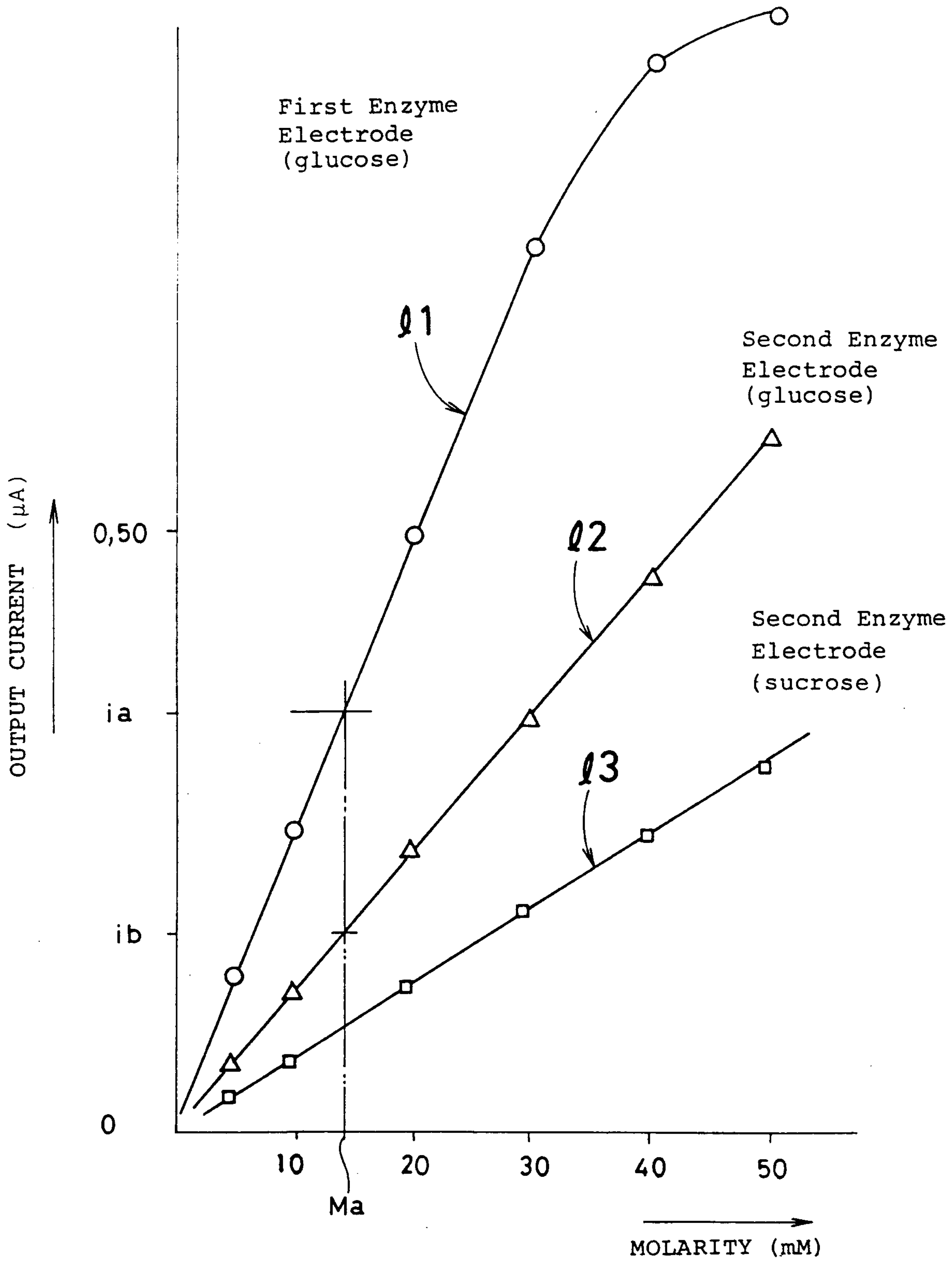
First Enzyme
Electrode
(glucose)
ℓ1
Second Enzyme
Electrode
(glucose)
ℓ2
Second Enzyme
Electrode
(sucrose)
ℓ3
OUTPUT CURRENT (μA)
0,50
ia
ib
0
10
20
30
40
50
Ma
MOLARITY (mM)

Fig. 6

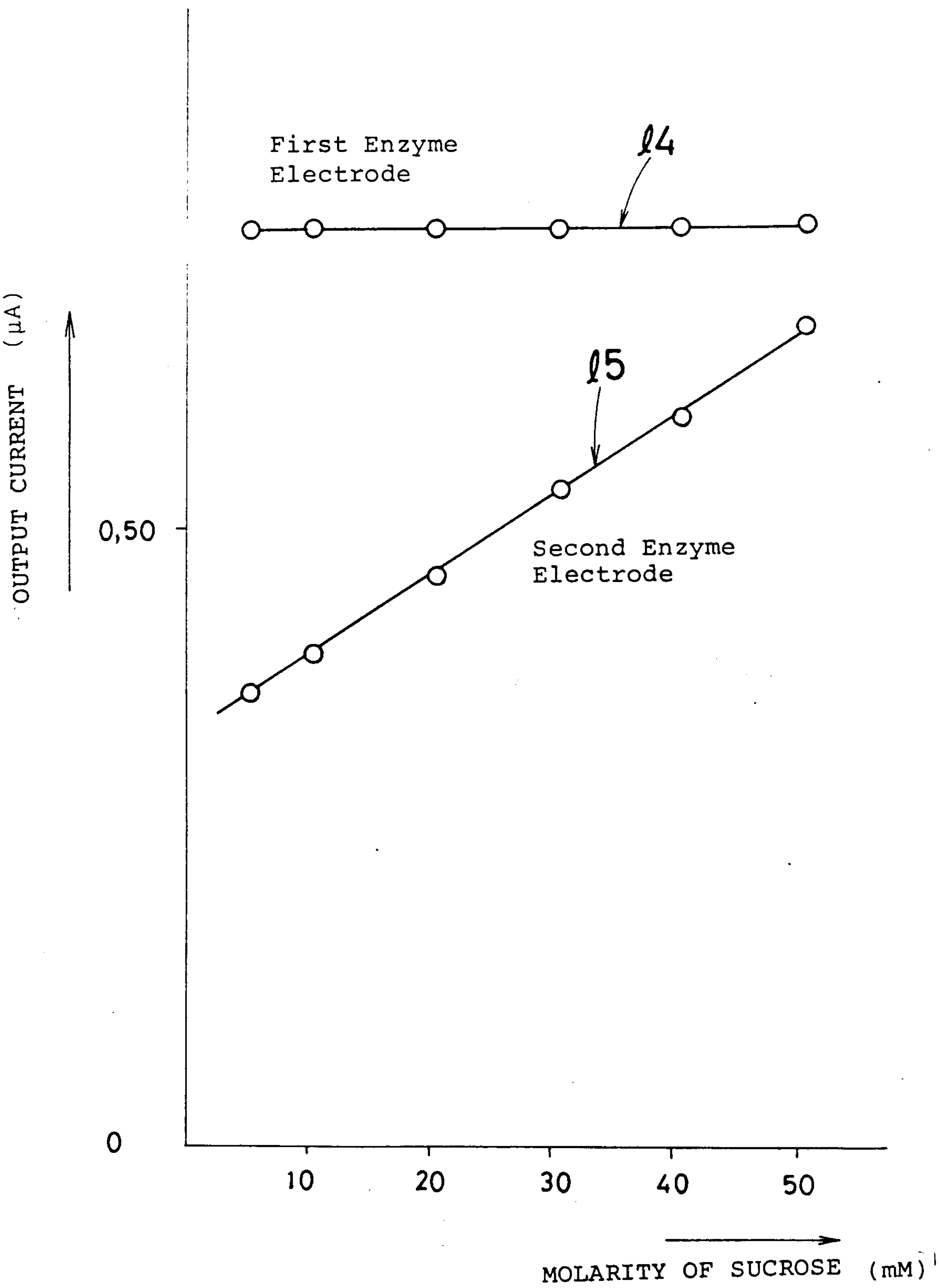
First Enzyme
Electrode
ℓ4
ℓ5
Second Enzyme
Electrode
OUTPUT CURRENT (μA)
0,50
0
10
20
30
40
50
MOLARITY OF SUCROSE (mM)

Fig.7

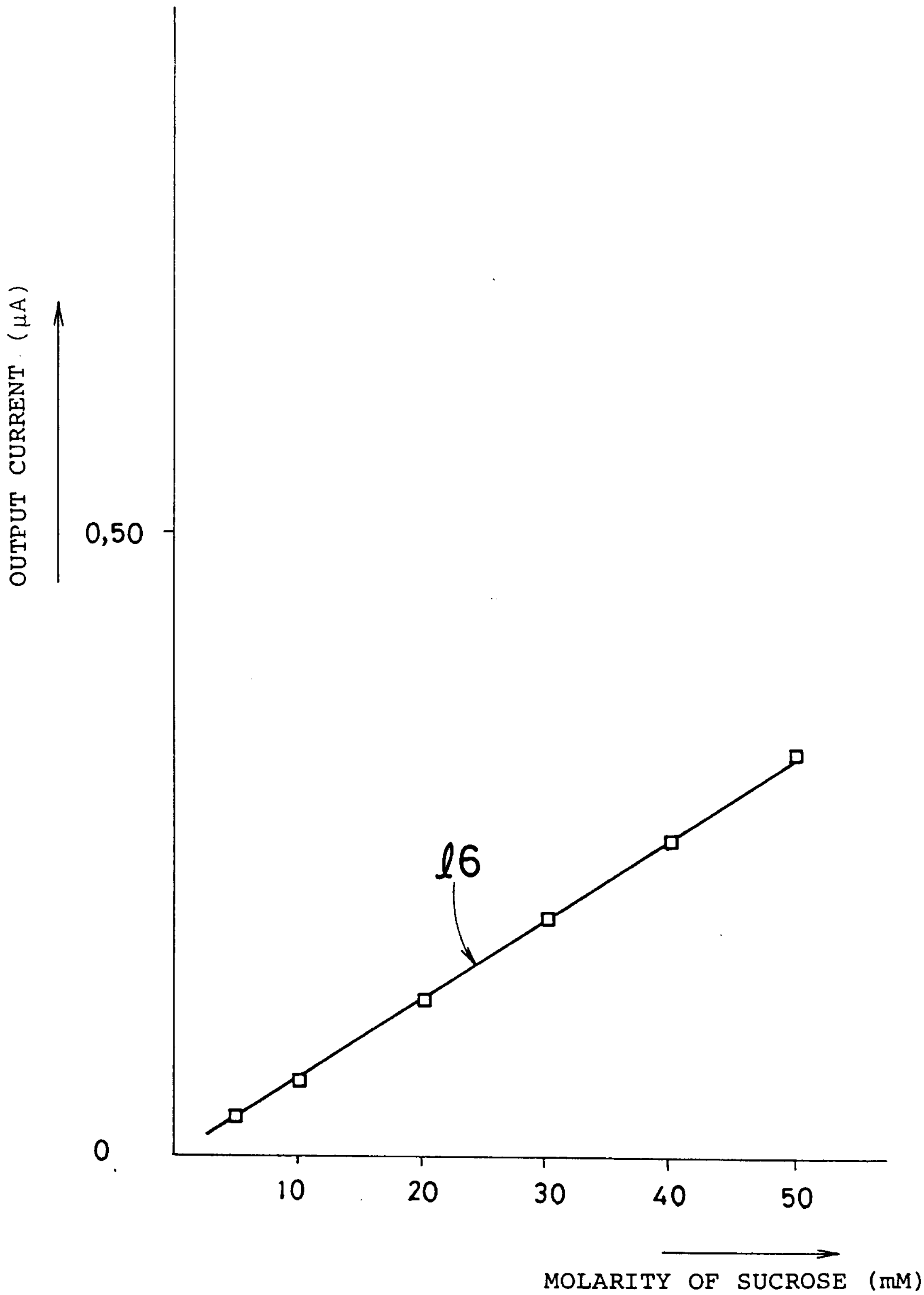
OUTPUT CURRENT (μA)
0,50
0
ℓ6
10
20
30
40
50
MOLARITY OF SUCROSE (mM)

Fig.8

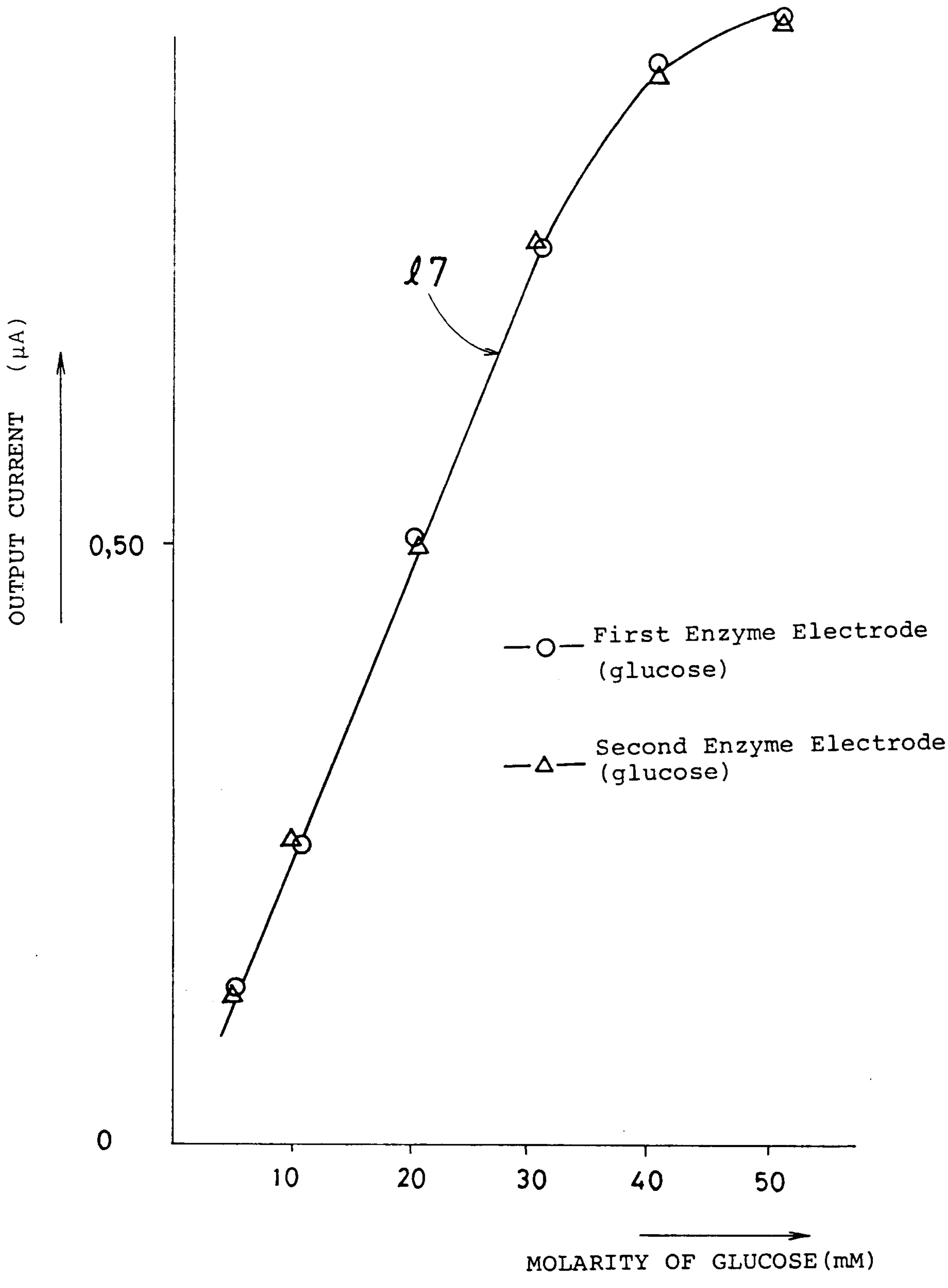
OUTPUT CURRENT (μA)
0,50
0
ℓ7
—○— First Enzyme Electrode (glucose)
—△— Second Enzyme Electrode (glucose)
10
20
30
40
50
MOLARITY OF GLUCOSE (mM)

Fig. 9

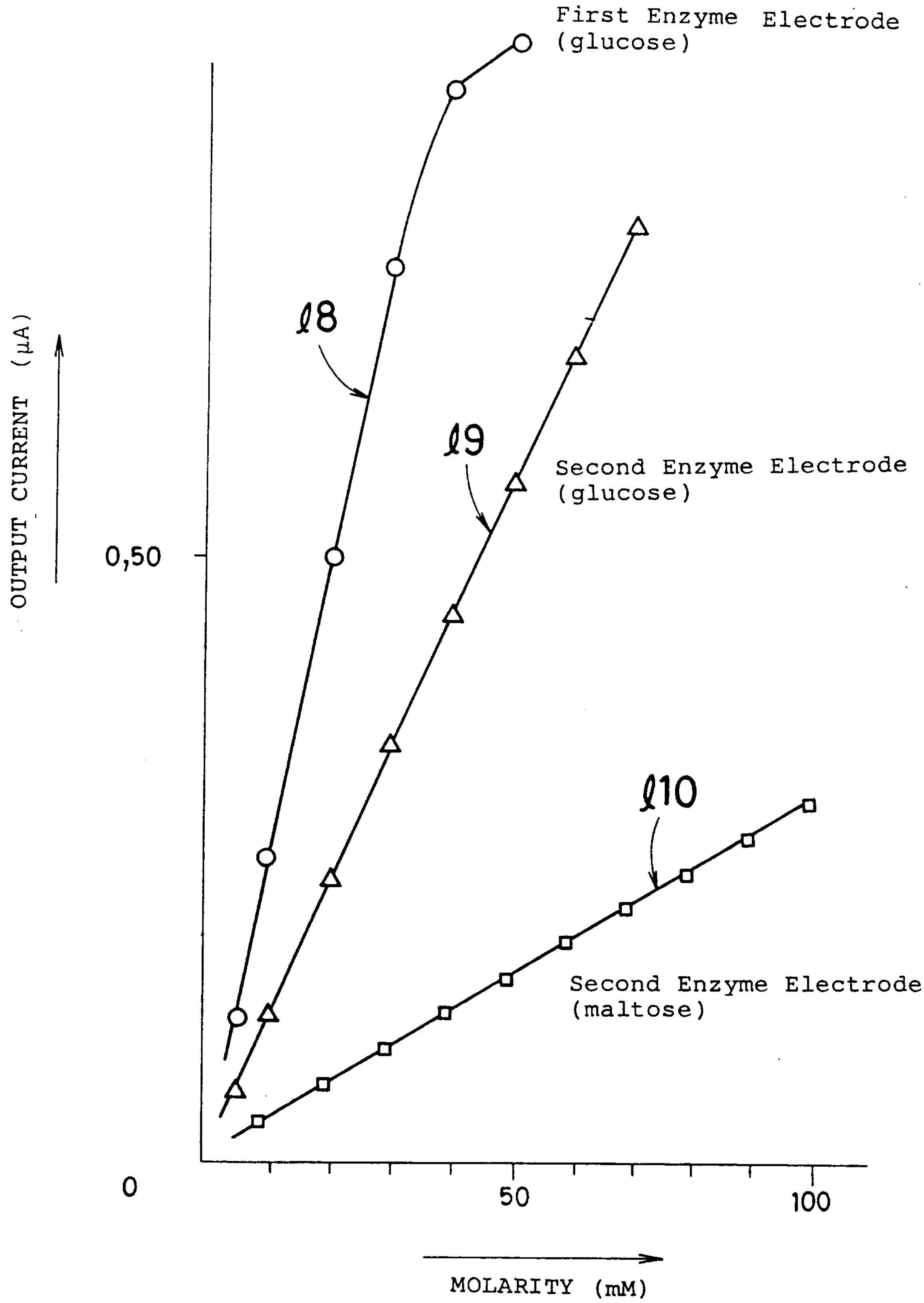
First Enzyme Electrode
(glucose)
ℓ8
ℓ9
Second Enzyme Electrode
(glucose)
ℓ10
Second Enzyme Electrode
(maltose)
OUTPUT CURRENT (μA)
0,50
0
50
100
MOLARITY (mM)

Fig.10

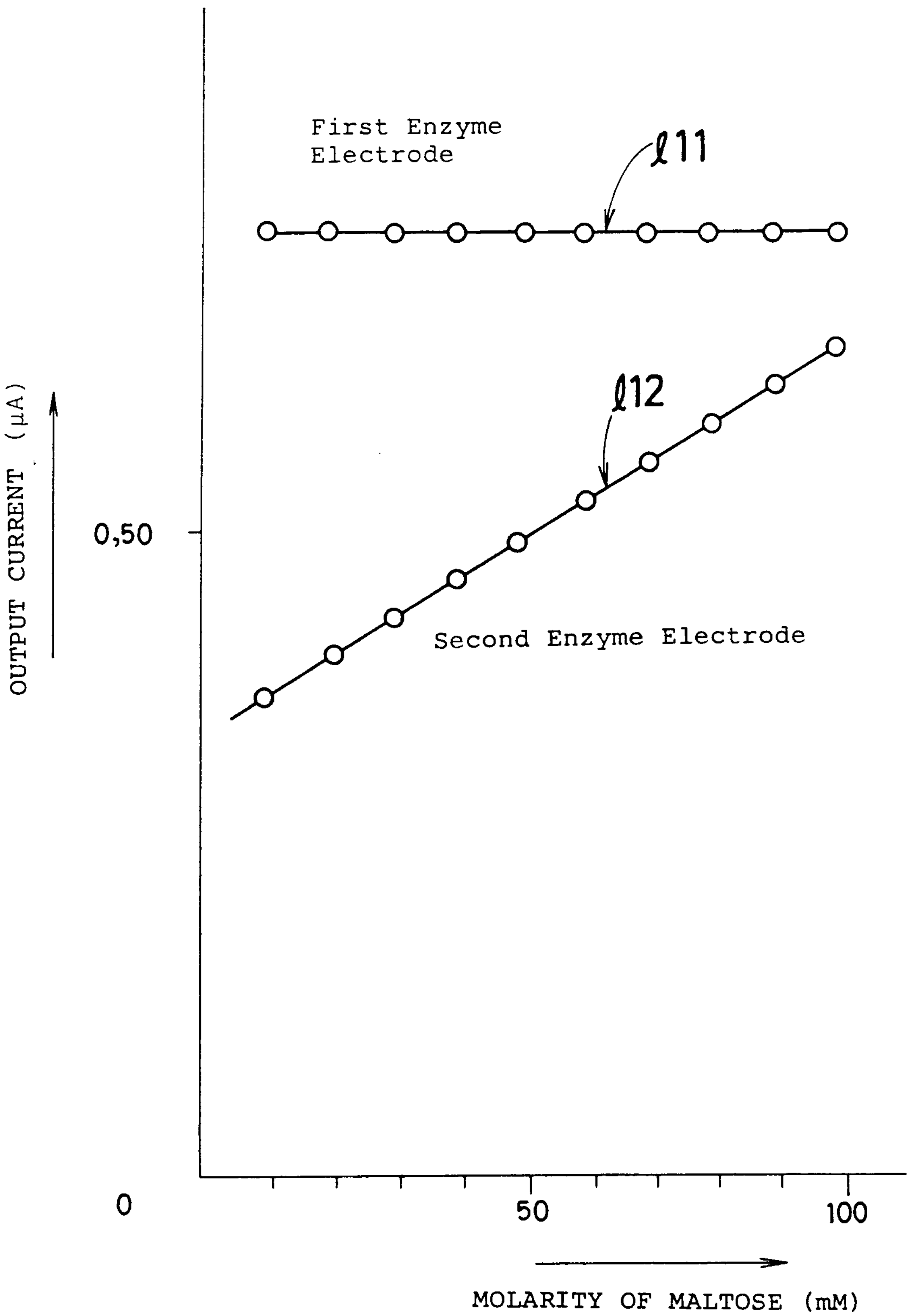
First Enzyme Electrode
ℓ11
ℓ12
Second Enzyme Electrode
OUTPUT CURRENT (μA)
0,50
0
50
100
MOLARITY OF MALTOSE (mM)

Fig.11

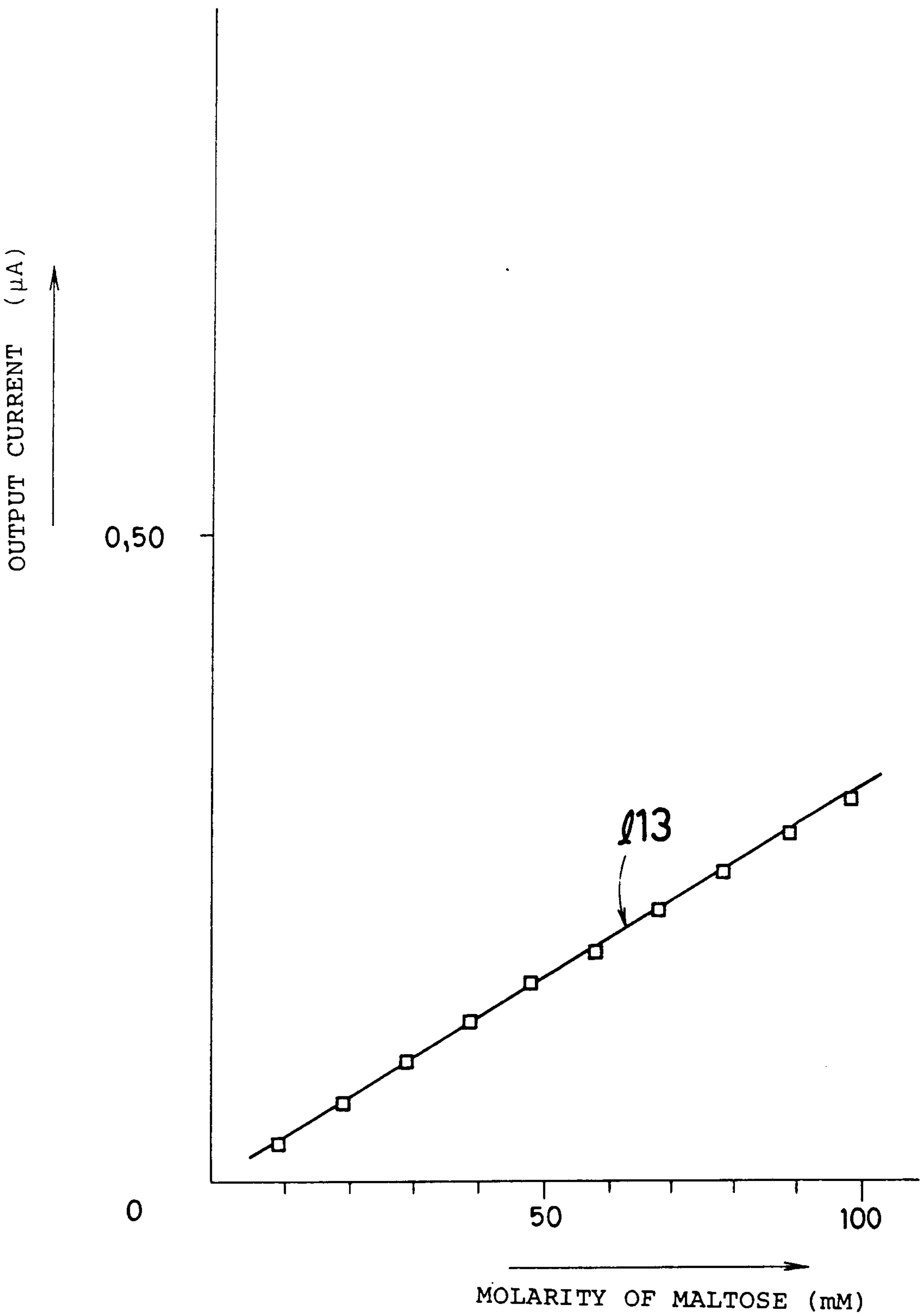

Fig.12

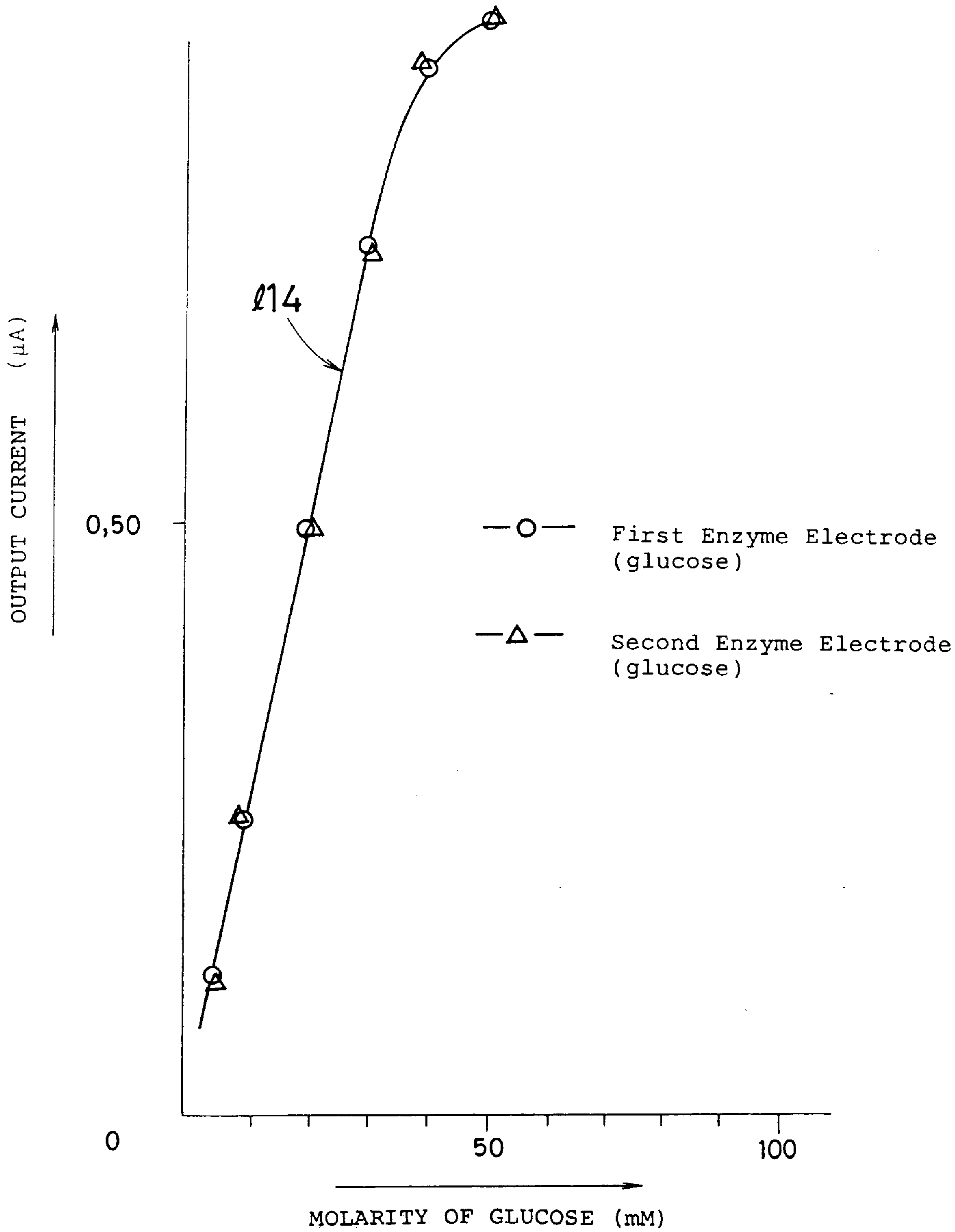
OUTPUT CURRENT (μA)
0,50
0
50
100
MOLARITY OF GLUCOSE (mM)
ℓ14
First Enzyme Electrode (glucose)
Second Enzyme Electrode (glucose)

Fig.13

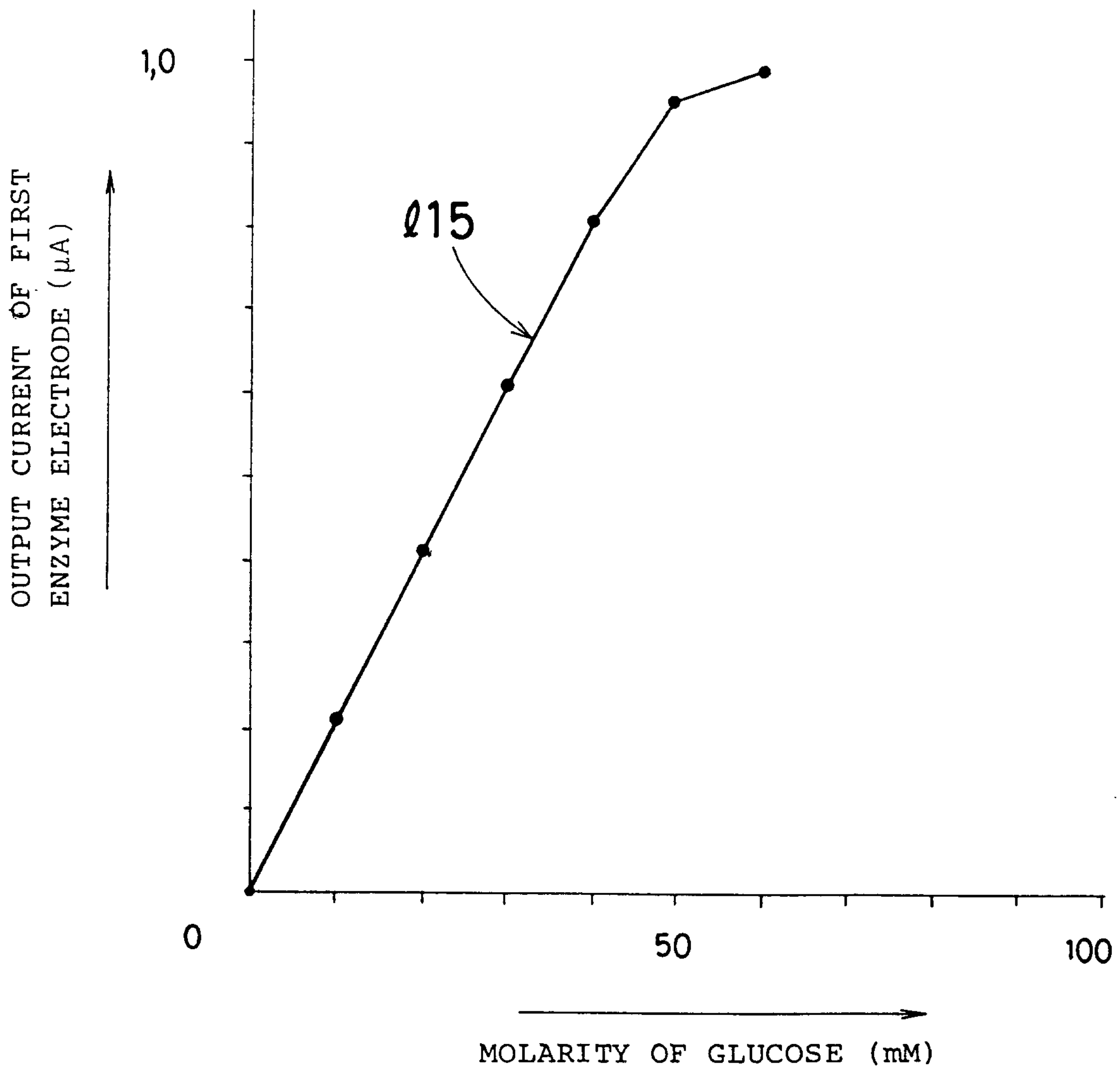
1,0
ℓ15
OUTPUT CURRENT OF FIRST ENZYME ELECTRODE (μA)
0
50
100
MOLARITY OF GLUCOSE (mM)

Fig.14

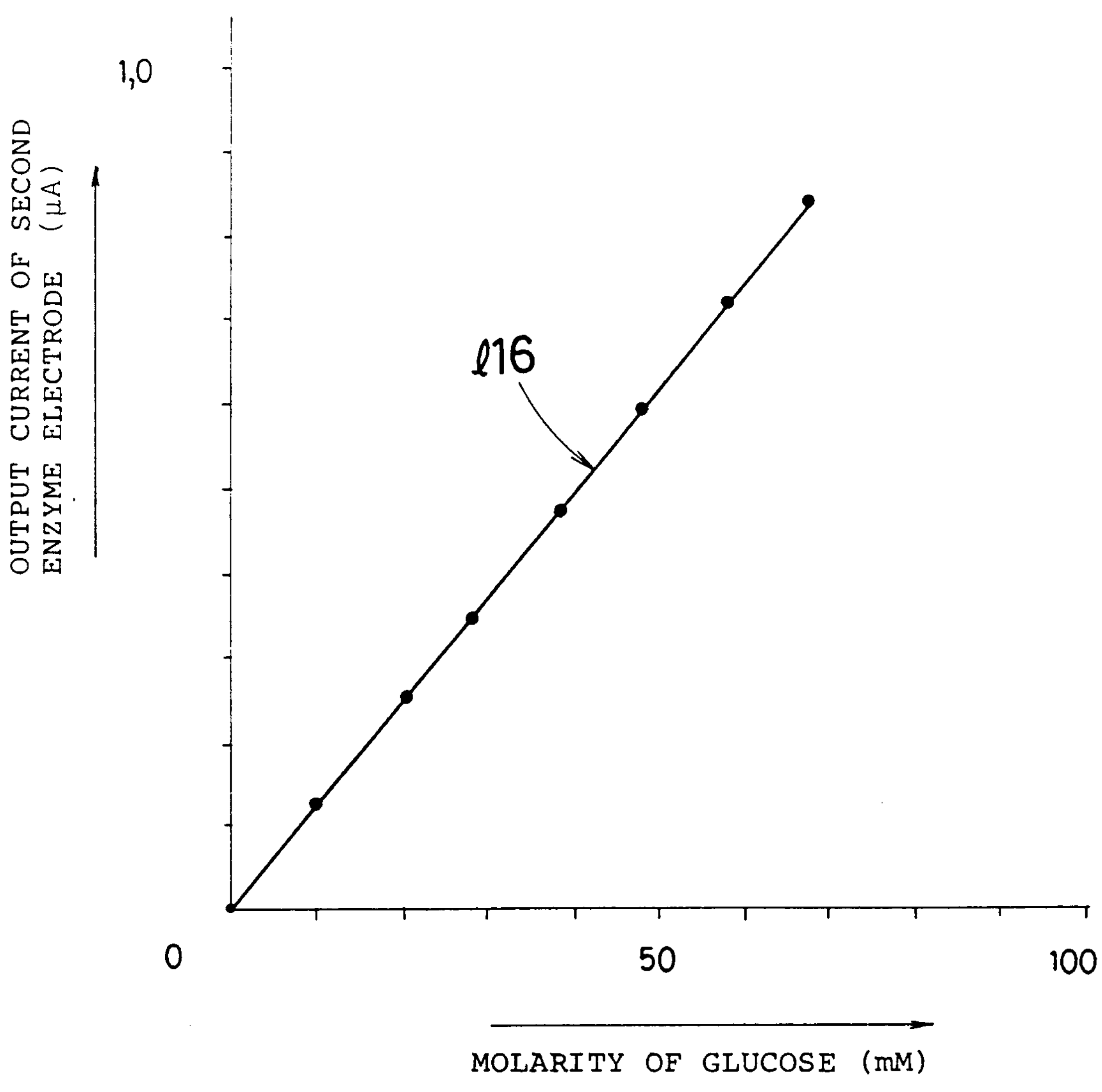

1,0
OUTPUT CURRENT OF SECOND ENZYME ELECTRODE (μA)
ℓ16
0
50
100
MOLARITY OF GLUCOSE (mM)

Fig.15

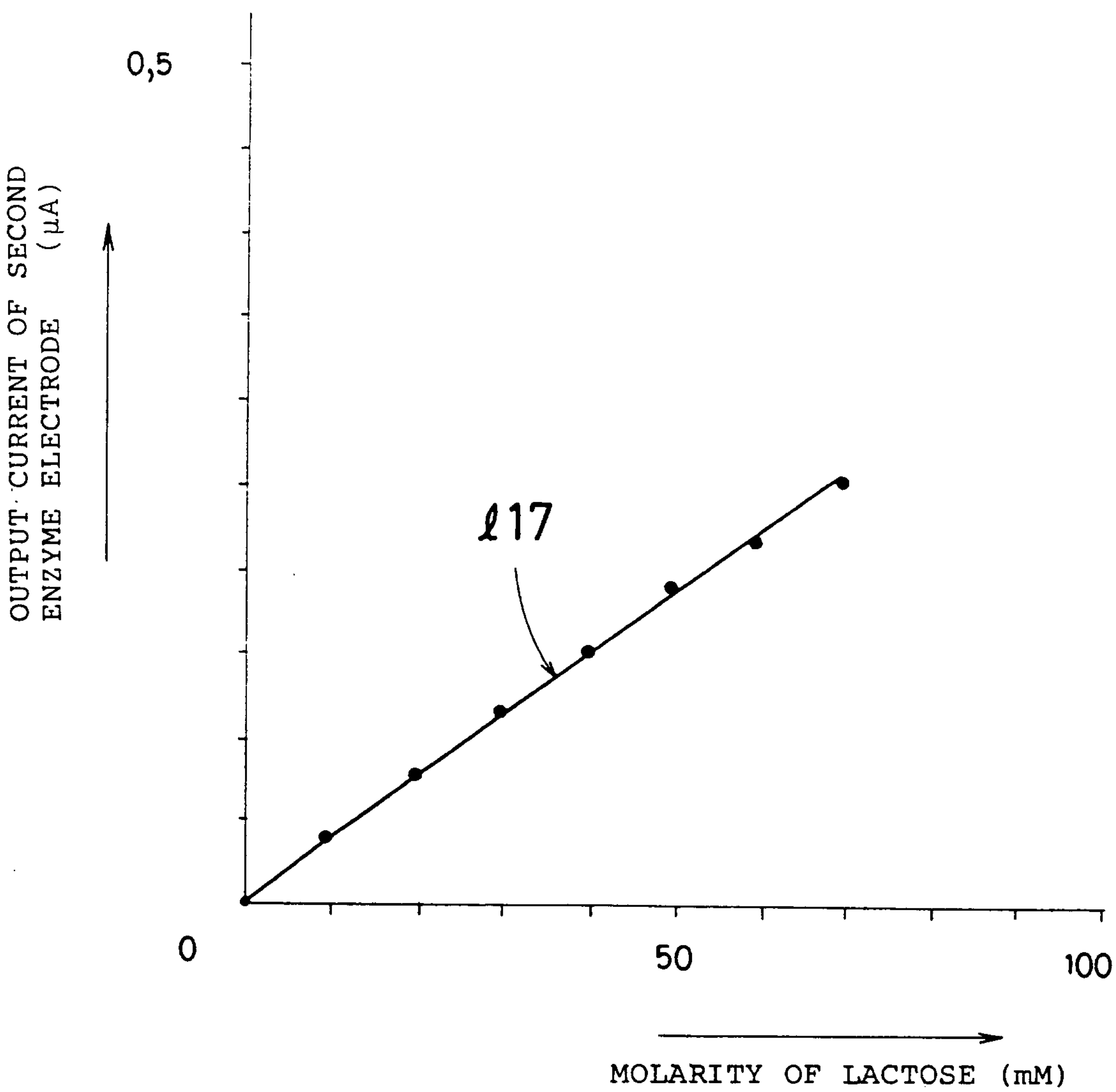
0,5
OUTPUT·CURRENT OF SECOND ENZYME ELECTRODE (μA)
ℓ17
0
50
100
MOLARITY OF LACTOSE (mM)

*Fig.16*

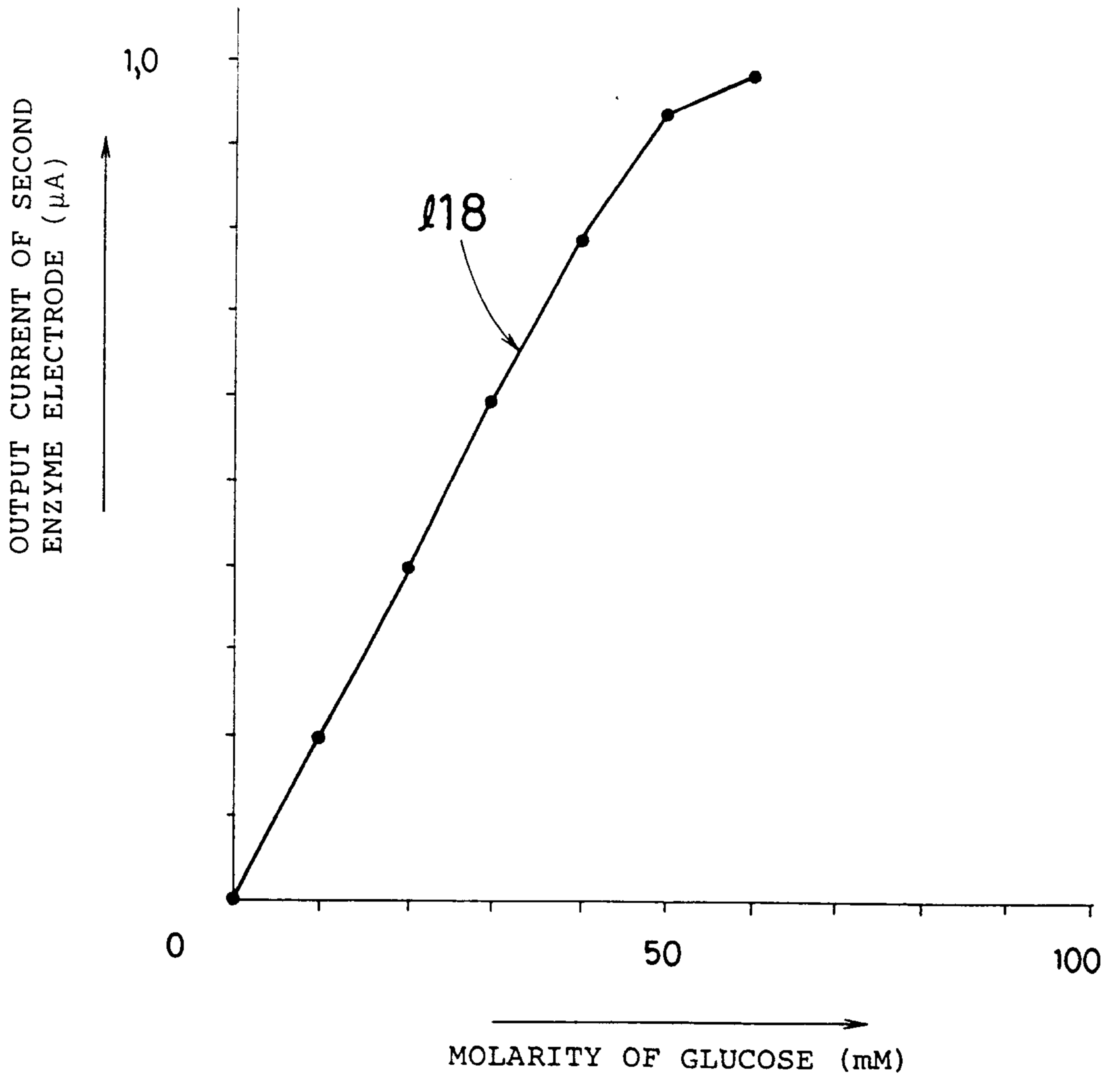

1,0
OUTPUT CURRENT OF SECOND ENZYME ELECTRODE (μA)
ℓ18
0
50
100
MOLARITY OF GLUCOSE (mM)